# EUROPEAN PATENT SPECIFICATION

(11) **EP 2 176 409 B1**
(45) Date of publication and mention of the grant of the patent: **04.01.2012**
(21) Application number: 08776602.8
(22) Date of filing: 10.07.2008
(51) Int. Cl.: C12N 15/10, C12Q 1/68, G01N 33/50

(54) **NUCLEIC ACID CONSTRUCT SYSTEMS CAPABLE OF DIAGNOSING OR TREATING A CELL STATE**
NUKLEINSÄUREKONSTRUKTSYSTEME MIT FÄHIGKEIT ZUR DIAGNOSE ODER BEHANDLUNG EINES ZELLZUSTANDS
SYSTÈMES DE PRODUITS DE CONSTRUCTION D'ACIDE NUCLÉIQUE POUVANT DIAGNOSTIQUER OU TRAITER UN ÉTAT CELLULAIRE

(30) Priority: 11.07.2007 US 929736 P; 28.12.2007 US 6193
(43) Date of publication of application: 21.04.2010
(73) Proprietor: YEDA RESEARCH AND DEVELOPMENT CO. LTD., 76100 Rehovot (IL)
(72) Inventor: NISSIM, Lior, 68190 Tel-aviv (IL); BAR-ZIV, Roy, 76100 Rehovot (IL)
(74) Representative: Fichter, Robert Arno
(86) International application number: PCT/IL2008/000963
(87) International publication number: WO 2009/007980

(56) References cited:
- WO-A-00/66707
- WO-A-01/25420
- WO-A-01/73105
- WO-A-01/83808
- WO-A-02/31201
- WO-A-95/20652
- WO-A2-01/96373
- NISSIM LIOR ET AL: "An autonomous system for identifying and governing a cell's state in yeast." PHYSICAL BIOLOGY SEP 2007, vol. 4, no. 3, September 2007 (2007-09), pages 154-163, XP020123687 ISSN: 1478-3975
- HUBSMAN M ET AL: "A novel approach for the identification of protein-protein interaction with integral membrane proteins" NUCLEIC ACIDS RESEARCH, OXFORD UNIVERSITY PRESS, SURREY, GB, vol. 29, no. 4, 15 February 2001 (2001-02-15), page E18, XP002237898 ISSN: 0305-1048
- BRODER Y C ET AL: "THE RAS RECRUITMENT SYSTEM, A NOVEL APPROACH TO THE STUDY OF PROTEIN-PROTEIN INTERACTIONS" CURRENT BIOLOGY, CURRENT SCIENCE, GB, vol. 8, no. 20, 1 October 1998 (1998-10-01), pages 1121-1124, XP000867036 ISSN: 0960-9822
- BARAK YOAV ET AL: "Matching fusion protein systems for affinity analysis of two interacting families of proteins: the cohesin-dockerin interaction." JOURNAL OF MOLECULAR RECOGNITION : JMR 2005 NOV-DEC, vol. 18, no. 6, November 2005 (2005-11), pages 491-501, XP002498643 ISSN: 0952-3499 cited in the application
- HANDELSMAN T ET AL: "Cohesin-dockerin interaction in cellulosome assembly: a single Asp-to-Asn mutation disrupts high-affinity cohesin-dockerin binding" FEBS LETTERS, ELSEVIER, AMSTERDAM, NL, vol. 572, no. 1-3, 13 August 2004 (2004-08-13), pages 195-200, XP004526241 ISSN: 0014-5793 cited in the application
- FIEROBE H P ET AL: "Design and production of active cellulosome chimeras. Selective incorporation of dockerin-containing enzymes into defined functional complexes." THE JOURNAL OF BIOLOGICAL CHEMISTRY 15 JUN 2001, vol. 276, no. 24, 15 June 2001 (2001-06-15), pages 21257-21261, XP002498644 ISSN: 0021-9258 cited in the application
- CHUANG HAN-YU ET AL: "Network-based classification of breast cancer metastasis." MOLECULAR SYSTEMS BIOLOGY 2007, vol. 3, 2007, page 140, XP002498645 ISSN: 1744-4292 cited in the application

### SEQUENCE LISTING

<110> Yeda Research And Development Co. Ltd.
   Nissim, Lior
   Bar-Ziv, Roy
<120> NUCLEIC ACID CONSTRUCT SYSTEMS CAPABLE OF DIAGNOSING OR TREATING A CELL STATE
<130> 43783
<150> US 60/929,736
   <151> 2007-07-11
<150> US 61/006,193
   <151> 2007-12-28
<160> 19
<170> PatentIn version 3.5
<210> 1
   <211> 817
   <212> DNA
   <213> Artificial sequence
<220>
   <223> Human derived CXCL1 promoter
<400> 1
   cctgcaggtt ccactgacgc taacactgga ttcagctttt gacactgata atctgttgcc      60
   accaaatgga aaacgtaaac aagatattct aagtgtggtt agagaatatg caacacaagg     120
   aacaagcaga acattcttct ctggaatctg acataatgga ctgtactttc acagacagca     180
   ctgatgttag atgtacgtga aataggctaa actgaaaata agaaaggctg aggcagagag     240
   gataatatag ctccagccta tctcccagca ccttgttaat ttctctcaat ctccagccac     300
   aaatccgaga cacaacgctc ttcctccaaa gaggtcgcgc cttctctgtg gtggttctca     360
   gggatccgcc ccagctcctt ctccgttccc agccccacac actgggatca ccaggcaccc     420
   aagatcccac ctctcaggtg gtatcttcag cgcaggctgc cactcagccc ccctccaggg     480
   atctggggca gaaggcgaat atcccagagt ctcagagtcc acaggagtta ctctgaaggg     540
   cgaggcgcgg gctgcatcag tggaccccca caccccaccc gcaccccaag cgctccaccc     600
   tgggggcggg gccgtcgcct tccttccgga ctcgggatcg atctggaact ccgggaattt     660
   ccctggcccg ggggctccgg gctttccagc cccaaccatg cataaaaggg gttcgcggat     720
   ctcggagagc cacagagccc gggccgcagg cacctcctcg ccagctcttc cgctcctctc     780
   acagccgcca gacccgcctg ctgagccccg gcgcgcc                              817
<210> 2
   <211> 1028
   <212> DNA
   <213> Artificial sequence
<220>
   <223> Human derived Histone-H2Al promoter
<400> 2
   cctgcaggtg agttaatagc tctgcgcgga gggaggggat ggagaggggg tccttgatcg      60
   cctcccaaca ttactgagct acatcacctg agcagtttct accccgattt ctgtaacgag     120
   cagtttttct cgttctgtgc cggcgcgcgc gcgcacacac acacacacac acacacacac     180
   acggctcaga ttcggccgca cccggcaacc gctagggtgc atggagacac attaggttac     240
   ataacccttc accgtgttcg aaaccctttc ttgatcgtgt gggtggctct gaaaagagcc      300
   tttgggttca ggacgccgag gaacgcctca cttggagctg gtgtacttgg tgacagcctt      360
   ggtgccctcg gacacggcgt gcttggccag ctcgccgggc agcagcaggc gaacggccgt      420
   ctgcacttcg cgggacgtga tggtggagcg cttgttgtag tgtgccaggc gggaggcctc      480
   gctggcgatg cgctcgaaga tgtcattgac gaaggagttc atgatgccca tggccttgga      540
   cgagatgccg gtgtcggggt gcacctgctt cagcaccttg tacacgtaga tagaatagct      600
   ctccttgcgg ccgcgcttgc gcttcttgcc gtccttcttc tgtgccttgg tgacagcctt      660
   tttagaaccc ttcttgggcg caggagccga tttggacggg tctggcatga tggctgagtc      720
   tctccaaaca gaaacgcgcg gcgctcggag taactctatt tgtacgtttt gtattcaaat      780
   gaaggctcag gatttgctca cttctgattg gatcaaacgt tgttctacgt catcgctggg      840
   aaaggaatac gcaaattagg agtgccaggt tctttttctg attggctacc atagccatcc      900
   aatcgaacgc cgcggtctag cctacctctg taccatacat aagggctcgc tggccttcac      960
   tgccctcttg tttttagtct cgcttttcgg ttgccgttgt cttttttcct tgactcggaa     1020
   ggcgcgcc                                                               1028
<210> 3
   <211> 966
   <212> DNA
   <213> Artificial sequence
<220>
   <223> Human derived MAGEA1 promoter
<400> 3
   cctgcaggca tgaaagtcag gaccctgagg ggtgaccgag ggtcccccaa ccccacgccc      60
   aaccccacta ccaccaacac gacgacttaa gccccggggc accactggca tccctccccc     120
   taccaccccc aatccctccc tttacgccac ccatccaaac atcttcacgc tcacccccag     180
   cccaagccag gcagaatccg gttccacccc tgctctcaac ccagggaagc ccaggtgccc     240
   agatgtgacg ccactgactt gagcattagt ggttagagag aagcgaggtt ttcggtctga     300
   ggggcggctt gagatcggtg gagggaagcg ggcccaggct ctgtaaggag gcaaggtgac     360
   atgctgaggg aggactgagg acccacttac cccagataga ggaccccaaa taatcccttc     420
   atgccagtcc tggaccatct ggtggtggac ttctcaggct gggccacccc cagccccctt    480
   gctgcttaaa ccactgggga ctctgaagtc agagctcgtg tgatcaggga agggctgctt     540
   aggagagggc agcgtccagg ctctgccaga catcatgctc aggattctca aggagggctg     600
   agggtcccta agaccccact cccgtgaccc aacccccact ccaatgctca ctcccgtgac     660
   ccaaccccct cttcattgtc attccaaccc ccaccccaca tcccccaccc catccctcaa    720
   ccctgatgcc catccgccca gccattccac cctcaccccc acccccaccc ccacgcccac     780
   tcccaccccc acccaggcag gatccggttc ccgccaggaa acatccgggt gcccggatgt     840
   gacgccactg acttgcgcat tgtggggcag agagaagcga ggtttccatt ctgagggacg     900
   gcgtagagtt cggccgaagg aacctgaccc aggctctgtg aggaggcaag gttttcaggg     960
   cgcgcc                                                                966
<210> 4
   <211> 1027
   <212> DNA
   <213> Artificial sequence
<220>
   <223> Human derived Myc promoter
<400> 4
   tctagatcta gacctgcagg gcccgagact gttgcaaacc ggcgccacag ggcgcaaagg 60
   ggatttgtct cttctgaaac ctggctgaga aattgggaac tccgtgtggg aggcgtgggg 120
   gtgggacggt ggggtacaga ctggcagaga gcaggcaacc tccctctcgc cctagcccag 180
   ctctggaaca ggcagacaca tctcagggct aaacagacgc ctcccgcacg gggccccacg 240
   gaagcctgag caggcggggc aggaggggcg gtatctgctg ctttggcagc aaattggggg 300
   actcagtctg ggtggaaggt atccaatcca gatagctgtg catacataat gcataataca 360
   tgactccccc caacaaatgc aatgggagtt tattcataac gcgctctcca agtatacgtg 420
   gcaatgcgtt gctgggttat tttaatcatt ctaggcatcg ttttcctcct tatgcctcta 480
   tcattcctcc ctatctacac taacatccca cgctctgaac gcgcgcccat taataccctt 540
   ctttcctcca ctctccctgg gactcttgat caaagcgcgg ccctttcccc agccttagcg 600
   aggcgccctg cagcctggta cgcgcgtggc gtggcggtgg gcgcgcagtg cgttctcggt 660
   gtggagggca gctgttccgc ctgcgatgat ttatactcac aggacaagga tgcggtttgt 720
   caaacagtac tgctacggag gagcagcaga gaaagggaga gggtttgaga gggagcaaaa 780
   gaaaatggta ggcgcgcgta gttaattcat gcggctctct tactctgttt acatcctaga 840
   gctagagtgc tcggctgccc ggctgagtct cctccccacc ttccccaccc tccccaccct 900
   ccccataagc gcccctcccg ggttcccaaa gcagagggcg tgggggaaaa gaaaaaagat 960
   cctctctcgc taatctccgc ccaccggccc tttataatgc gagggtctgg acggctgagg 1020
   gcgcgcc                                                           1027
<210> 5
   <211> 834
   <212> DNA
   <213> Artificial sequence
<220>
   <223> Human derived SSX1 promoter
<400> 5
   cctgcagggt agccagatca tggctcactg caacctcgta ctcctgggct caagctatcc 60
   tcctacctca gcctcctgag taacggacta caggcacacc accccacctc gctaatttta 120
   tttatttttt ttgtagagaa aagagacagg gtattgctct gttgcccagg gtggggtgca 180
   gtggcatgat catggctcac tgcaacctct gcctcccagg ttcaagtgat cctccagctg 240
   tggcctccct aagtgctggg attacaaccg tgagccgccg caccggccca aatttcttac 300
   gtcactacag agttcctagg aaaaaatccc atacctgaaa aagatagaaa ctgacaggaa 360
   ggatttgaga tgatgacctg cttcatatac actccttatt aaaactggat aacaatgcac 420
   caccgaggag gtggggaggg ataggaaaaa tgaaaagaga aaatcagcgc atgcgtactc 480
   tgatttggga agactccaaa gagaaaatca gagcatgcgt actctgaact tggagtagcc 540
   aatcccaggg gatgctttag gcgggaaagt cagagtttct gcctccattt tgagaaggtt 600
   ctgtccctag agcctagact gatagacccc acatcagctt ggcttgtccc gcctactgtt 660
   ctgacttctg attggccaga tggagttcac taactgccct gattggtcca tcatcctgga 720
   gcaatgacat tgcagaatat tttctcctcc tccagccaca ctttgtcacc aactgctgcc 780
   aactcgccac cactgctgcc gacctcgcaa ccactgcttt gtctctggcg cgcc 834
<210> 6
   <211> 1076
   <212> DNA
   <213> Artificial sequence
<220>
   <223> Human derived Wisp1 promoter
<400> 6
   acggcttctg accaatgagc agagacggca aacagcaaat gcgttaccgt ggctttcacg 60
   tgtcatgaag gtgatgctgt aacctgagcc tcagagctca ttttatttgt gaattcctat 120
   catctctaga tgggtggact gaaagccacc tctttcgctt gcagctaggt agaggggagt 180
   gcaagagggc aaatgaaaac cagaagtagg gttgggcgca atgcctcacg cctgtaatcc 240
   cagcactttg ggaggctgat gtgggcggat tacctgaggt caggagtttg agaccagcct 300
   ggccaacatg gtgaaacctc gtctctacta aaaatacaaa aatcagctgg gtgtggtggc 360
   acatgcctgt aatctcagct attcaggagg ctgaggcagg agaattgctt gagcctggga 420
   gacagaggtt gcagtgagca gagatcgcgc cactgcactc cagcctggcc gacagagcga 480
   gactctgtct caaaaaaaaa aaaaaaaaaa aaaaaaaaga aaagaaaaga aaaagaaaaa 540
   gaaaaggaaa aagaaaacca gaagtaggag cagcctgaag aaatgacagg gagttgattt 600
   cccactggga gccctctcaa agcccacaca cccgcctgcc tggggtaaca gtatctcctc 660
   ggacatcctg caccctccca tgctcccccc tccctacagt agtgaaagac ctaggcagga 720
   tgaccccagc tcctctgtga gaatttcaca ccctagtgtg aagtcatagc cttgtaactt 780
   tccctttaag aactgtcaga gctggggagg ctggccaagc tcaggctgga ggtggggaca 840
   gagggaagaa agaaaaaaaa aaaagagaga gaggcaggaa aagttttttc agaggaaaat 900
   gcagggtttg tccttcaccc tgacgtcaga tcttgcttta ataaaacccc ccaagggctg 960
   cggaagaggc atatctggtg ctcctgatgg gccggccagt ctgggcccag ctcccccgag 1020
   aggtggtcgg atcctctggg ctgctcggtc gatgcctgtg ccactgacgt ccaggc 1076
<210> 7
   <211> 1065
   <212> DNA
   <213> Artificial sequence
<220>
   <223> Human derived MMP3 promoter
<400> 7
   taaaagtttt acaaaatgtc ttcctctgaa tatgtttaga gtcttgcatt caagcattta      60
   ttatacacca ataatgtgag caacacttta cttgacaaag aaacagaaaa gaaaggaaag     120
   gaagaaaaca gaagagcatg aagagaaaat ttaggatgga ttctgttctt caacttcaaa     180
   gcatctgcta atttgaattt agggaggagg ggaaaaggtt gaaagagaat aagacatgtg     240
   tagaagacaa ggacagagag aatttcagtc cggtaagcaa tgtaattcat ttcaattcta     300
   caactattta tggagcagct acgtgggccc atcacccatt aataaattgg ttacagaatt     360
   aaaaccaacc caaagggaat atacttcctt ctttttcaca gaccctcttt gttctattct     420
   gcccatgagg ttttcctcct caagaaccag caaatccaac gacagtcaat agcaggcatt     480
   acaaatcaga ttcagaaaaa taaatcaccc cttctaaatt tcttctagat attatctttt     540
   atgttttgag tataattgta tatagtatag actatagcta tgtatgtaca ctttccactt     600
   acatctttta tttgctttta taatgtgttt cttaaaataa aactgctttt agaagttctg     660
   cacaattctg atttttacca agtcaaccta cttcttctct caaaaggaca aacataaatt     720
   gtctagtgaa ttccagtcaa tttttccaga agaaaaaaaa tgctccagtt ttctcctcta     780
   ccaagacagg aagcacttcc tggagattaa tcactgtgtt gccttgcaaa attgggaagg     840
   ttgagagaaa ttagtaaagt aggttgtatc atcctacttt gaatttggaa tgtttggaaa     900
   tggtcctgct gccatttgga tgaaagcaag gatgagtcaa gctgcgggtg atccaaacaa     960
   acactgtcac tctttaaaag ctgcgctccc gaggttggac ctacaaggag gcaggcaaga    1020
   cagcaaggca tagagacaac atagagctaa gtaaagccag tggaa                    1065
<210> 8
   <211> 1087
   <212> DNA
   <213> Artificial sequence
<220>
   <223> Human derived IL1B promoter
<400> 8
   gagactctgt ctcaaaaaaa aaaaaaaagt gttatgatgc agacctgtca aagaggcaaa      60
   ggagggtgtt cctacactcc aggcactgtt cataacctgg actctcattc attctacaaa     120
   tggagggctc ccctgggcag taccctggag caggcacttt gctggtgtct cggttaaaga     180
   gaaactgata actcttggtt ggtattacca agagatagag tctcagatgg atattcttac     240
   agaaacaata ttccactttt cagagttcac caaaaaatca ttttaggcag agctcatctg     300
   gcattgatct ggttcatcca tgagattggc tagggtaaca gcacctggtc ttgcagggtt     360
   gtgtgagctt atctccaggg ttgccccaac tccgtcagga gcctgaaccc tgcataccgt     420
   atgttctctg ccccagccaa gaaaggtcaa ttttctcctc agaggctcct gcaattgaca     480
   gagagctcct gaggcagaga acagcaccca aggtagagac ccacaccctc aatacagaca     540
   gggagggcta ttggcccttc attgtaccca tttatccatc tgtaagtggg aagattccta     600
   aacttaagta caaagaagtg aatgaagaaa agtatgtgca tgtataaatc tgtgtgtctt     660
   ccactttgtc ccacatatac taaatttaaa cattcttcta acgtgggaaa atccagtatt     720
   ttaatgtgga catcaactgc acaacgattg tcaggaaaac aatgcatatt tgcatggtga     780
   tacatttgca aaatgtgtca tagtttgcta ctccttgccc ttccatgaac cagagaatta     840
   tctcagttta ttagtcccct cccctaagaa gcttccacca atactctttt cccctttcct     900
   ttaacttgat tgtgaaatca ggtattcaac agagaaattt ctcagcctcc tacttctgct     960
   tttgaaagcc ataaaaacag cgagggagaa actggcagat accaaacctc ttcgaggcac    1020
   aaggcacaac aggctgctct gggattctct tcagccaatc ttcattgctc aagtgtctga    1080
   agcagcc                                                              1087
<210> 9
   <211> 696
   <212> DNA
   <213> Artificial sequence
<220>
   <223> YFP protein coding sequence
<400> 9
   agcggcgccc tgctgttcca cggcaagatc ccctacgtgg tggagatgga gggcgatgtg      60
   gatggccaca ccttcagcat ccgcggtaag ggctacggcg atgccagcgt gggcaaggtg     120
   gatgcccagt tcatctgcac caccggcgat gtgcccgtgc cctggagcac cctggtgacc     180
   accctgacct acggcgccca gtgcttcgcc aagtacggcc ccgagctgaa ggatttctac     240
   aagagctgca tgcccgatgg ctacgtgcag gagcgcacca tcaccttcga gggcgatggc     300
   aatttcaaga cccgcgccga ggtgaccttc gagaatggca gcgtgtacaa tcgcgtgaag     360
   ctgaatggcc agggcttcaa gaaggatggc cacgtgctgg gcaagaatct ggagttcaat     420
   ttcacccccc actgcctgta catctggggc gatcaggcca atcacggcct gaagagcgcc     480
   ttcaagatct gccacgagat cgccggcagc aagggcgatt tcatcgtggc cgatcacacc     540
   cagatgaata cccccatcgg cggcggcccc gtgcacgtgc ccgagtacca ccacatgagc     600
   taccacgtga agctgagcaa ggatgtgacc gatcaccgcg ataatatgag cctgacggag     660
   accgtgcgcg ccgtggattg ccgcaagacc tacctg                               696
<210> 10
   <211> 1132
   <212> DNA
   <213> Artificial sequence
<220>
   <223> TK1 protein coding sequence
<400> 10
   atggcttcgt acccctgcca tcaacacgcg tctgcgttcg accaggctgc gcgttctcgc     60
   ggccatagca accgacgtac ggcgttgcgc cctcgccggc agcaagaagc cacggaagtc    120
   cgcctggagc agaaaatgcc cacgctactg cgggtttata tagacggtcc tcacgggatg    180
   gggaaaacca ccaccacgca actgctggtg gccctgggtt cgcgcgacga tatcgtctac    240
   gtacccgagc cgatgactta ctggcaggtg ctgggggctt ccgagacaat cgcgaacatc    300
   tacaccacac aacaccgcct cgaccagggt gagatatcgg ccggggacgc ggcggtggta    360
   atgacaagcg cccagataac aatgggcatg ccttatgccg tgaccgacgc cgttctggct    420
   cctcatatcg ggggggaggc tgggagctca catgccccgc ccccggccct caccctcatc    480
   ttcgaccgcc atcccatcgc cgccctcctg tgctacccgg ccgcgcgata ccttatgggc    540
   agcatgaccc cccaggccgt gctggcgttc gtggccctca tcccgccgac cttgcccggc    600
   acaaacatcg tgttgggggc ccttccggag gacagacaca tcgaccgcct ggccaaacgc    660
   cagcgccccg gcgagcggct tgacctggct atgctggccg cgattcgccg cgtttacggg    720
   ctgcttgcca atacggtgcg gtatctgcag ggcggcgggt cgtggcggga ggattgggga    780
   cagctttcgg ggacggccgt gccgccccag ggtgccgagc cccagagcaa cgcgggccca    840
   cgaccccata tcggggacac gttatttacc ctgtttcggg cccccgagtt gctggccccc    900
   aacggcgacc tgtacaacgt gtttgcctgg gccttggacg tcttggccaa acgcctccgt    960
   cccatgcacg tctttatcct ggattacgac caatcgcccg ccggctgccg ggacgccctg   1020
   ctgcaactta cctccgggat ggtccagacc cacgtcacca cccccggctc cataccgacg   1080
   atctgcgacc tggcgcgcac gtttgcccgg gagatggggg aggctaactg ag            1132
<210> 11
   <211> 210
   <212> DNA
   <213> Artificial sequence
<220>
   <223> VP16-AD
<400> 11
   atgaagctac tgtcttctat cgaacaagca tgcccaaaaa agaagagaaa ggtagatgaa     60
   ttcccgggga tctcgacggc ccccccgacc gatgtcagcc tgggggacga gctccactta    120
   gacggcgagg acgtggcgat ggcgcatgcc gacgcgctag acgatttcga tctggacatg    180
   ttgggggacg gggattcccc gggtccggga                                     210
<210> 12
   <211> 441
   <212> DNA
   <213> Artificial sequence
<220>
   <223> GAL4-BD
<400> 12
   atgaagctac tgtcttctat cgaacaagca tgcgatattt gccgacttaa aaagctcaag      60
   tgctccaaag aaaaaccgaa gtgcgccaag tgtctgaaga acaactggga gtgtcgctac     120
   tctcccaaaa ccaaaaggtc tccgctgact agggcacatc tgacagaagt ggaatcaagg     180
   ctagaaagac tggaacagct atttctactg atttttcctc gagaagacct tgacatgatt     240
   ttgaaaatgg attctttaca ggatataaaa gcattgttaa caggattatt tgtacaagat     300
   aatgtgaata aagatgccgt cacagataga ttggcttcag tggagactga tatgcctcta     360
   acattgagac agcatagaat aagtgcgaca tcatcatcgg aagagagtag taacaaaggt     420
   caaagacagt tgactgtatc g                                               441
<210> 13
   <211> 420
   <212> DNA
   <213> Artificial sequence
<220>
   <223> Coh2
<400> 13
   ggtggtagta gaaattggca aagttacggg atctgttgga actacagttg aaatacctgt      60
   atatttcaga ggagttccat ccaaaggaat agcaaactgc gactttgtgt tcagatatga     120
   tccgaatgta ttggaaatta tagggataga tcccggagac ataatagttg acccgaatcc     180
   taccaagagc tttgatactg caatatatcc tgacagaaag ataatagtat tcctgtttgc     240
   ggaagacagc ggaacaggag cgtatgcaat aactaaagac ggagtatttg caaaaataag     300
   agcaactgta aaatcaagtg ctccgggcta tattactttc gacgaagtag gtggatttgc     360
   agataatgac ctggtagaac agaaggtatc atttatagac ggtggtgtta acgtttaagg     420
<210> 14
   <211> 216
   <212> DNA
   <213> Artificial sequence
<220>
   <223> DocS
<400> 14
   tctactaaat tatacggcga cgtcaatgat gacggaaaag ttaactcaac tgacgctgta      60
   gcattgaaga gatatgtttt gagatcaggt ataagcatca acactgacaa tgccgatttg     120
   aatgaagacg gcagagttaa ttcaactgac ttaggaattt tgaagagata tattctcaaa     180
   gaaatagata cattgccgta caagaactaa actagt                              216
<210> 15
   <211> 124
   <212> DNA
   <213> Artificial sequence
<220>
   <223> PESTubiquitination target sequence
<400> 15
   agccatggct tcccgccggc ggtggcggcg caggatgatg gcacgctgcc catgtcttgt      60
   gcccaggaga gcgggatgga ccgtcaccct gcagcctgtg cttctgctag gatcaatgtg     120
   tagg                                                                  124
<210> 16
   <211> 104
   <212> DNA
   <213> Artificial sequence
<220>
   <223> 5xGA1,4-BS
<400> 16
   acggagtact gtcctccgag cggagtactg tcctccgact cgagcggagt actgtcctcc      60
   gatcggagta ctgtcctccg cgaattccgg agtactgtcc tccg                      104
<210> 17
   <211> 1654
   <212> DNA
   <213> Artificial sequence
<220>
   <223> Luciferase reporter coding sequence
<400> 17
   atggaagacg ccaaaaacat aaagaaaggc ccggcgccat tctatccgct ggaagatgga      60
   accgctggag agcaactgca taaggctatg aagagatacg ccctggttcc tggaacaatt     120
   gcttttacag atgcacatat cgaggtggac atcacttacg ctgagtactt cgaaatgtcc     180
   gttcggttgg cagaagctat gaaacgatat gggctgaata caaatcacag aatcgtcgta     240
   tgcagtgaaa actctcttca attctttatg ccggtgttgg gcgcgttatt tatcggagtt     300
   gcagttgcgc ccgcgaacga catttataat gaacgtgaat tgctcaacag tatgggcatt     360
   tcgcagccta ccgtggtgtt cgtttccaaa aaggggttgc aaaaaatttt gaacgtgcaa     420
   aaaaagctcc caatcatcca aaaaattatt atcatggatt ctaaaacgga ttaccaggga     480
   tttcagtcga tgtacacgtt cgtcacatct catctacctc ccggttttaa tgaatacgat     540
   tttgtgccag agtccttcga tagggacaag acaattgcac tgatcatgaa ctcctctgga     600
   tctactggtc tgcctaaagg tgtcgctctg cctcatagaa ctgcctgcgt gagattctcg     660
   catgccagag atcctatttt tggcaatcaa atcattccgg atactgcgat tttaagtgtt     720
   gttccattcc atcacggttt tggaatgttt actacactcg gatatttgat atgtggattt     780
   cgagtcgtct taatgtatag atttgaagaa gagctgtttc tgaggagcct tcaggattac     840
   aagattcaaa gtgcgctgct ggtgccaacc ctattctcct tcttcgccaa aagcactctg      900
   attgacaaat acgatttatc taatttacac gaaattgctt ctggtggcgc tcccctctct      960
   aaggaagtcg gggaagcggt tgccaagagg ttccatctgc caggtatcag gcaaggatat     1020
   gggctcactg agactacatc agctattctg attacacccg agggggatga taaaccgggc     1080
   gcggtcggta aagttgttcc attttttgaa gcgaaggttg tggatctgga taccgggaaa    1140
   acgctgggcg ttaatcaaag aggcgaactg tgtgtgagag gtcctatgat tatgtccggt     1200
   tatgtaaaca atccggaagc gaccaacgcc ttgattgaca aggatggatg gctacattct     1260
   ggagacatag cttactggga cgaagacgaa cacttcttca tcgttgaccg cctgaagtct     1320
   ctgattaagt acaaaggcta tcaggtggct cccgctgaat tggaatccat cttgctccaa     1380
   caccccaaca tcttcgacgc aggtgtcgca ggtcttcccg acgatgacgc cggtgaactt     1440
   cccgccgccg ttgttgtttt ggagcacgga aagacgatga cggaaaaaga gatcgtggat     1500
   tacgtcgcca gtcaagtaac aaccgcgaaa aagttgcgcg gaggagttgt gtttgtggac     1560
   gaagtaccga aaggtcttac cggaaaactc gacgcaagaa aaatcagaga gatcctcata     1620
   aaggccaaga agggcggaaa gatcgccgtg taat                                 1654
<210> 18
   <211> 957
   <212> DNA
   <213> Artificial sequence
<220>
   <223> Murine derived MyoDl
<400> 18
   atggagcttc tatcgccgcc actccgggac atagacttga caggccccga cggctctctc       60
   tgctcctttg agacagcaga cgacttctat gatgacccgt gtttcgactc accagacctg      120
   cgcttttttg aggacctgga cccgcgcctg gtgcacatgg gagccctcct gaaaccggag      180
   gagcacgcac acttccctac tgcggtgcac ccaggcccag gcgctcgtga ggatgagcat      240
   gtgcgcgcgc ccagcgggca ccaccaggcg ggtcgctgct tgctgtgggc ctgcaaggcg      300
   tgcaagcgca agaccaccaa cgctgatcgc cgcaaggccg ccaccatgcg cgagcgccgc      360
   cgcctgagca aagtgaatga ggccttcgag acgctcaagc gctgcacgtc cagcaacccg      420
   aaccagcggc tacccaaggt ggagatcctg cgcaacgcca tccgctacat cgaaggtctg      480
   caggctctgc tgcgcgacca ggacgccgcg ccccctggcg ccgctgcctt ctacgcacct      540
   ggaccgctgc ccccaggccg tggcagcgag cactacagtg gcgactcaga tgcatccagc      600
   ccgcgctcca actgctctga tggcatgatg gattacagcg gccccccaag cggcccccgg     660
   cggcagaatg gctacgacac cgcctactac agtgaggcgg cgcgcgagtc caggccaggg      720
   aagagtgcgg ctgtgtcgag cctcgactgc ctgtccagca tagtggagcg catctccaca      780
   gacagccccg ctgcgcctgc gctgcttttg gcagatgcac caccagagtc gcctccgggt      840
   ccgccagagg gggcatccct aagcgacaca gaacagggaa cccagacccc gtctcccgac      900
   gccgcccctc agtgtcctgc aggctcaaac cccaatgcga tttatcaggt gctttga         957
<210> 19
   <211> 447
   <212> DNA
   <213> Artificial sequence
<220>
   <223> Murine derived ID1
<400> 19
   atgaaggtcg ccagtggcag tgccgcagcc gctgcaggcc ctagctgttc gctgaaggcg      60
   ggcaggacag cgggcgaggt ggtacttggt ctgtcggagc aaagcgtggc catctcgcgc     120
   tgcgctggga cgcgcctgcc cgccttgctg gacgagcagc aggtgaacgt cctgctctac     180
   gacatgaacg gctgctactc acgcctcaag gagctggtgc ccaccctgcc ccagaaccgc     240
   aaagtgagca aggtggagat cctgcagcat gtaatcgact acatcaggga cctgcagctg     300
   gagctgaact cggagtctga agtcgggacc accggaggcc ggggactgcc tgtccgcgcc     360
   ccgctcagca ccctgaacgg cgagatcagt gccttggcgg ccgaggcggc atgtgttcca     420
   gccgacgatc gcatcttgtg tcgctga                                         447

## Description

### FIELD AND BACKGROUND OF THE INVENTION

The present invention relates to a nucleic acid construct system according to claim 1 as well as a method of Identifying an agent capable of reversing a disease phenotype of a mammalian cell according to claim 12.

The present invention relates to a nucleic acid construct system which serves as an autonomous molecular computer and, to the use of same for the diagnosis and treatment of a cell state.

Physicians are required to make many medical decisions ranging from, for example, whether and when a patient is likely to experience a medical condition to how a patient should be treated once the patient has been diagnosed with the condition. Determining an appropriate course of treatment for a patient may increase the patient's chances for, for example, survival and/or recovery. Similarly, predicting the occurrence of an event advantageously allows individuals to plan for the event. For example, predicting whether a patient is likely to experience occurrence (e.g., recurrence) of a disease may allow a physician to recommend an appropriate course of treatment for that patient,

Physicians rely heavily on their expertise and training to treat, diagnose and predict the occurrence of medical conditions as well as on electronic computers for information concerning many medical applications.

The state of a cell can be diagnosed by monitoring the activity patterns of its various pathways. In certain cases it is possible to identify the state of the cell by the activity level of a single gene [Surana, U., et al., 1991. 65(1): p. 145-61]. For example, it was shown that the transcription level of a single gene can be used for reliable identification of cell cycle-phase [Spellman, P.T., et al., Mol Biol Cell, 1998. 9(12): p. 3273-97]. Nevertheless, in most cases it is essential to monitor the activity of several genes in order to accurately determine a cell-state. Thus, for example, a physician may rely on the ex-vivo analysis of expression patterns using DNA arrays to determine average states of cell populations. In addition, *in-vivo* measurements, for example by real-time microscopy, make it possible for a physician to diagnose a cell state at a single cell resolution. However, these methods rely on non-autonomous cell state analysis, which precludes the possibility of altering the state in vivo in real time.

In recent years there has been significant interest in exploring the possibilities of biological computation for such purposes. Such computers, using biological molecules as input data and biologically active molecules as outputs, could produce a system for "logical" control of biological processes - see for example Mao et al, Nature 407, 493-496 (2000); Sakamoto et al, Biosystems, 52, 81-91 (1999); Sakamoto et al, Science 288, 1223-1226 (2000); Benenson et al, Nature 414, 430-434 (2001); and Benenson et al., PNAS USA 100, 219102196 (2003).

Biomolecular computers hold the promise of direct computational analysis of biological information in its native biomolecular form, eschewing its conversion into an electronic representation. Recently this capability was shown to afford direct recognition and analysis of molecular disease indicators, providing *in vitro* disease diagnosis, which in turn was coupled to the programmed release of the biologically active molecule. In this work, autonomous state determination and response were demonstrated through the use of a molecular computer based on DNA/RNA hybridization and digestion [Benenson, Y., Gil, B., Ben-Dor, U., Adar, R. & Shapiro, E. (2004) Nature 429, 423-429]. However, schemes for autonomous diagnosis using molecular computation are yet to be implemented in living cells.

In addition, it has been demonstrated that engineered gene circuits can be interfaced with natural ones to obtain cells that respond to biological signals in a predetermined way. For example, in one case bacterial bio-film formation was coupled to DNA damage response [Kobayashi, H., et al., Proc Natl Acad Sci U S A, 2004. 101(22): p. 8414-9], and in another, cell killing was coupled to a transgenic quorum-sensing system [You, L., et al., Nature, 2004. 428(6985): p. 868-71].

Biomolecular computers comprise a further advantage in that unlike traditional computers, they can have direct access to a patient's biochemistry and thus are able to affect the fate of living cells in real-time. This is particularly advantageous in situations of phenotypic and genotypic importance, such as metabolism, proliferation or apoptosis.

A biomolecular computer would also have the significant advantage of being able to use internal energy resources, like ATP molecules, rather than being dependent on external or rechargeable energy sources.

Biomolecular computers capable of diagnosing cell states based on the yeast two hybrid system are known in the art.

The yeast-based two-hybrid system (Fields and Song (1989) Nature 340:245) has been traditionally used for elucidating protein-protein binding in cells. This system utilizes chimeric genes and detects protein-protein interactions via the activation of reporter-gene expression. Reporter-gene expression occurs as a result of reconstitution of a functional transcription factor caused by the association of fusion proteins encoded by the chimeric genes. Typically, polynucleotides encoding two-hybrid proteins are constructed and introduced into a yeast host cell. The first hybrid protein consists of the yeast Gal4 DNA-binding domain fused to a polypeptide sequence of a known protein (often referred to as the "bait"). The second hybrid protein consists of the Gal4 activation domain fused to a polypeptide sequence of a second protein (often referred to as the "prey"). Binding between the two-hybrid proteins reconstitutes the Gal4 DNA-binding domain with the Gal4 activation domain, which leads to the transcriptional activation of a reporter gene (e.g., lacZ or HIS3), which is operably linked to a Gal4 binding site.

U.S. Patent No. 6,479,289 teaches mammalian two-hybrid systems for elucidating protein-protein binding in mammalian cells.

U.S. Patent No. 6,787,321 teaches a mammalian two-hybrid system for diagnosing a diseased cell based on its ability to degrade a metabolic product which is comprised in the first hybrid protein.

Autonomous systems for cancer therapy have already been developed. These, however, were based on a single gene input and as such were limited in their implementation. This is because although a single-gene input was sufficient to discriminate cancer cells from normal ones in certain tissues, it was not sufficient for all tissues and it was also limited to particular cancers [Ohana, P., et al., 2002. 98(5): p. 645-50; Fellig, Y., et al., J Clin Pathol, 2005, 58(10): p. 1064-8; Ariel, I., et al., Mol Pathol, 2000. 53(6): p. 320-3]. This is a result of the fact that single-gene input is, in many cases, not sufficient for efficient selection.

"Nissim et at [Physical Biology Sep 2007, Vol. 4, No. 3, pages 154-163 teaches a system that measures the expression of several genes and integrates their activities into a single output in order to detect states of interest in a living cell.

International Patent Application WO 02131201 teaches a method for screening for an agent that modulates the ability of a cell to accumulate or to degrade a metabolic product, which involves providing a mammalian cell comprising a nucleic acid encoding a peptide binding site and an effector gene, and a first and second chimeric protein, contacting the cell with a test agent and detecting an alteration of expression of the effector gene.

International Patent Application WO 01/83808 teaches a nucleic acid construct system comprising a first nucleic acid construct including a polynucleotide encoding a polypeptide which is capable of Interacting with a plasmalemma of a cell in which It Is expressed and a second nucleic acid construct including a polynucleotide encoding a cytoplasmic Ras mutant fused to a polypeptide.

International Patent Application WO 00/66707 teaches an Isolated nucleic acid encoding a thiamine transporter which is used for research, diagnosis and treatment of thiamine responsive megaloblastic anemia (TRMA) syndrome.

International Patent Application WO 01/25420 discloses a method for detecting an interaction between a first and second test protein at variable sensitivities comprising (a) providing a host cell which expresses detectable gene product; (b) providing the host cell with a first interacting molecule comprising a DNA binding region and a second interacting molecule, comprising a region capable of transcriptional activation, wherein the host cell has the capacity to regulate amounts of the first or second interacting molecules; (c) regulating the amounts of the first or second interacting molecules so the detectable reporter gene is activated; and (d) determining the extent to which the detectable reporter gene has been activated.

International Patent Application WO 01/96373 teaches a fluorescent timer protein for monitoring the activity of a promoter, determining the age of a protein, identifying an agent that modulates the activity of a promoter and in enriching a population of cells comprising a fluorescent timer protein".

Thus, there remains a widely recognized need for, and it would be highly advantageous to have, a molecular computing unit capable of integrating more than one signal.

The nucleic acid construct system of the present invention is defined in claim 1 and the method of identifying an agent capable of reversing a disease phenotype of a mammalian cell of the present invention is defined in claim 12.

According to an aspect of some embodiments of the present invention there is provided a nucleic acid construct system, comprising:
(i) a first nucleic acid construct comprising a first polynucleotide, the first polynucleotide comprising a first nucleic acid sequence encoding a first expression product, the first nucleic acid sequence being operably linked to a first inducible mammalian transcriptional regulatory sequence; and
(ii) a second nucleic acid construct comprising a second polynucleotide, the second polynucleotide comprising a second nucleic acid sequence encoding a second expression product, the second nucleic acid sequence being operably linked to a second inducible mammalian transcriptional regulatory sequence,
wherein the first mammalian inducible transcriptional regulatory sequence and the second mammalian inducible transcriptional regulatory sequence are regulated by a metabolic state or a pathological state.

According to some embodiments of the invention, the nucleic acid construct system further comprises a third nucleic acid construct comprising a third polynucleotide, the third polynucleotide comprising a third nucleic acid sequence encoding a reporter polypeptide, operably linked to a promoter, wherein an activity of the promoter is regulated by binding of at least one of the first and the second expression product.

According to another aspect of some embodiments of the present invention there is provided a method of diagnosing a disease or a metabolic state, the method comprising expressing the nucleic acid construct system of the present invention in at least one mammalian cell, wherein a change in expression of the reporter polypeptide is indicative of the disease or metabolic state.

According to an aspect of some embodiments of the present invention there is provided a method of treating a disease, the method comprising ex vivo expressing the nucleic acid construct system of the present invention, in at least one cell of a subject in need thereof, thereby treating the disease.

According to an aspect of some embodiments of the present invention there is provided a method of identifying an agent capable of reversing a disease phenotype of a mammalian cell, the method comprising,
(a) expressing the nucleic acid construct system of the present invention in the mammalian cell;
(b) contacting the mammalian cell with the agent;
(c) measuring a level of detectable moiety following (b) and optionally prior to (b), wherein a reversion of phenotype is indicative of an agent capable of reversing a diseased phenotype of a mammalian cell.

According to an aspect of some embodiments of the present invention there is provided a nucleic acid construct, comprising:
(i) a first polynucleotide, the first polynucleotide comprising a first nucleic acid sequence encoding a first expression product, the first nucleic acid sequence being operably linked to a first inducible mammalian transcriptional regulatory sequence;and
(ii) a second polynucleotide comprising a second nucleic acid sequence encoding a second expression product, the second nucleic acid sequence being operably linked to a second inducible mammalian transcriptional regulatory sequence,
wherein the first mammalian inducible transcriptional regulatory sequence and the second mammalian inducible transcriptional regulatory sequence are regulated by a metabolic state or a pathological state.

According to some embodiments of the invention, the reporter polypeptide comprises a detectable moiety.

According to some embodiments of the invention, the reporter polypeptide comprises a therapeutic polypeptide.

According to some embodiments of the invention, the first expression product and the second expression product are capable of binding to form a transcriptional regulator of the promoter.

According to some embodiments of the invention, the transcriptional regulator is an activator of the promoter.

According to some embodiments of the invention, the transcriptional regulator is an inhibitor of the promoter.

According to some embodiments of the invention, the first inducible mammalian transcriptional regulatory sequence and the second inducible mammalian transcriptional regulatory sequence are non-identical and each independently selected from a cell cycle responsive mammalian transcriptional regulatory sequence.

According to some embodiments of the invention, when the first inducible mammalian transcriptional regulatory sequence is a CXCL1 regulatory sequence the second inducible mammalian transcriptional regulatory sequence is an MMP3 or an IL1β regulatory sequence.

According to some embodiments of the invention, the first inducible mammalian transcriptional regulatory sequence is an MMP3 regulatory sequence and the second inducible mammalian transcriptional regulatory sequence is an IL1β regulatory sequence.

According to some embodiments of the invention, the first inducible mammalian transcriptional regulatory sequence is an E2F1 regulatory sequence and the second inducible mammalian transcriptional regulatory sequence is a RAD51 regulatory sequence.

According to some embodiments of the invention, the first inducible mammalian transcriptional regulatory sequence is an SHC1 regulatory sequence and the second inducible mammalian transcriptional regulatory sequence is an VEGFβ regulatory sequence.

According to some embodiments of the invention, the first expression product is a polypeptide capable of activating the promoter.

According to some embodiments of the invention, the second expression product is a polynucleotide capable of down-regulating an expression of the first expression product or the reporter polypeptide.

According to some embodiments of the invention, the polynucleotide capable of down-regulating an expression of the first expression product is an RNA silencing oligonucleotide.

According to some embodiments of the invention, the first polynucleotide and/or the second polynucleotide further comprises a nucleic acid sequence encoding a degradation tag.

According to some embodiments of the invention, the second inducible mammalian transcriptional regulatory sequence is a tumor suppressor gene inducible mammalian transcriptional regulatory sequence.

According to some embodiments of the invention, the tumor suppressor gene inducible mammalian transcriptional regulatory sequence is selected from the group consisting of a p21 inducible regulatory sequence, a p16 inducible regulatory sequence, a p14 inducible regulatory sequence and a p53 inducible regulatory sequence.

According to some embodiments of the invention, when the tumor suppressor gene inducible mammalian transcriptional regulatory sequence is p21, the first inducible mammalian transcriptional regulatory sequence is E2F1.

According to some embodiments of the invention, when the first expression product is DOC2, the second expression product is Coh2.

According to some embodiments of the invention, the disease is cancer.

According to some embodiments of the invention, the disease is a metabolic disorder.

According to some embodiments of the invention, the therapeutic polypeptide comprises a cytotoxic or apoptotic activity.

According to some embodiments of the invention, the nucleic construct further comprises a third polynucleotide, the third polynucleotide comprising a third nucleic acid sequence encoding a reporter polypeptide, operably linked to a promoter, wherein an activity of the promoter is regulated by binding of at least one of the first and the second expression product.
Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. . In case of conflict, the patent specification, including definitions, will control.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG.1 is an illustration of an example of normal and aberrant expression of cell-cycle genes in normal and cancer cells respectively.

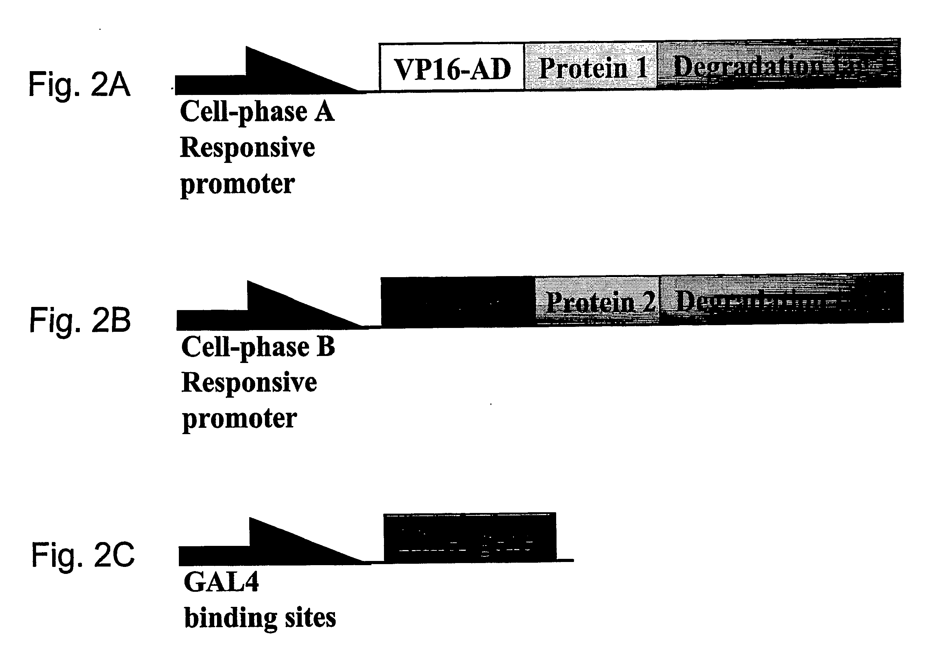

FIGs. 2A-C is an example of a nucleic acid construct system according to an embodiment of the present invention.
FIGs. 3A-L are maps of exemplary constructs of the present invention.
FIGs. 4A-E are maps of exemplary constructs of the present invention.
FIGs. 5A-B are bar graphs illustrating the calibration of the activity of input promoters. Mean activities of human promoters (in fluorescence units) were measured in (Figure 5A) WI38/T/R/G cells and (Figure 5B) 293T cells, using auxiliary plasmids pPromoter-Au (Figure 4A). DNA delivery was performed by transfection with FuGENE-HD reagent (Roche). This calibration was used to determine the activity level generated by each promoter as an input to the present system.

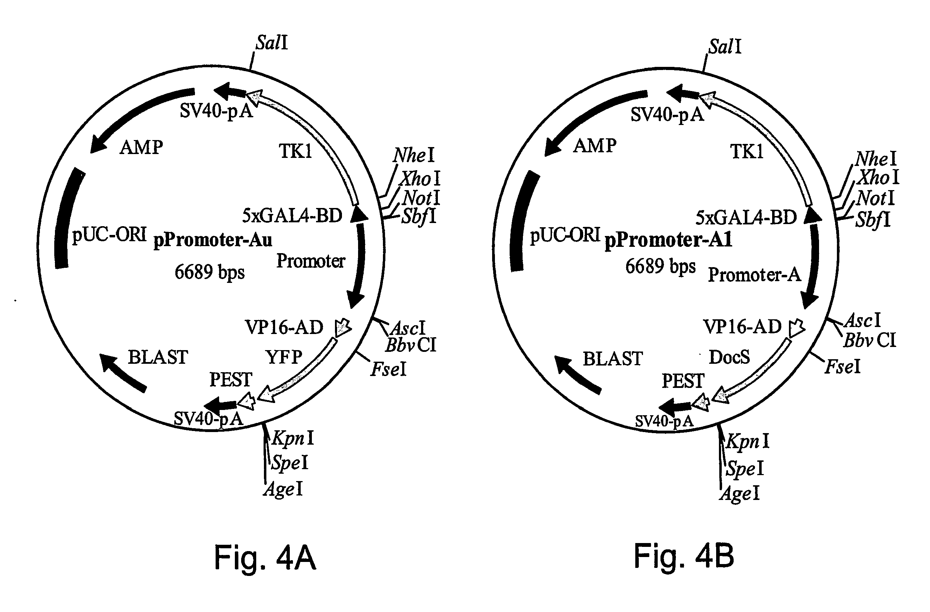

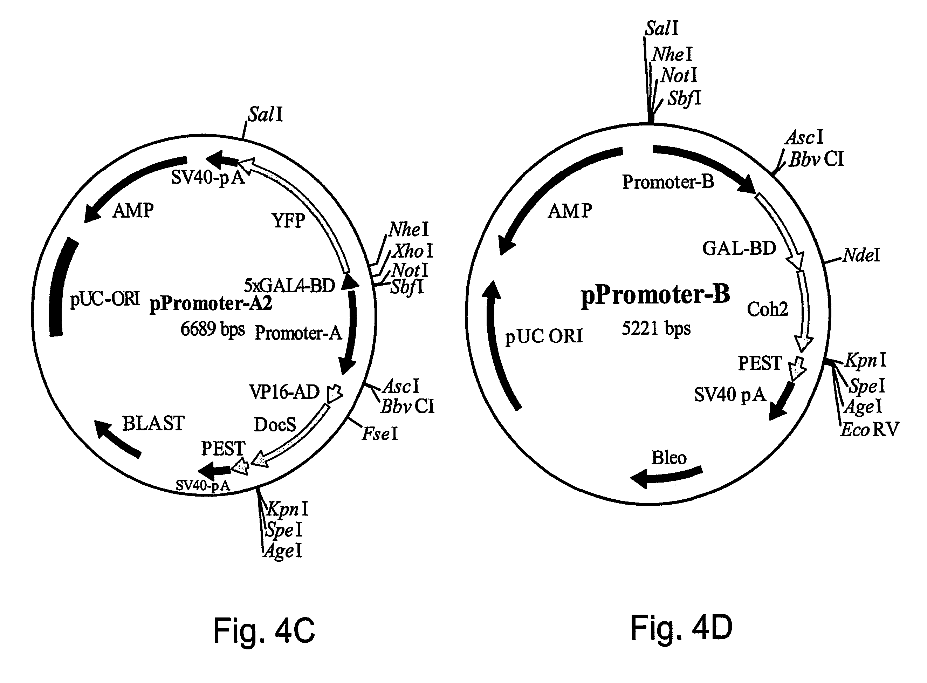

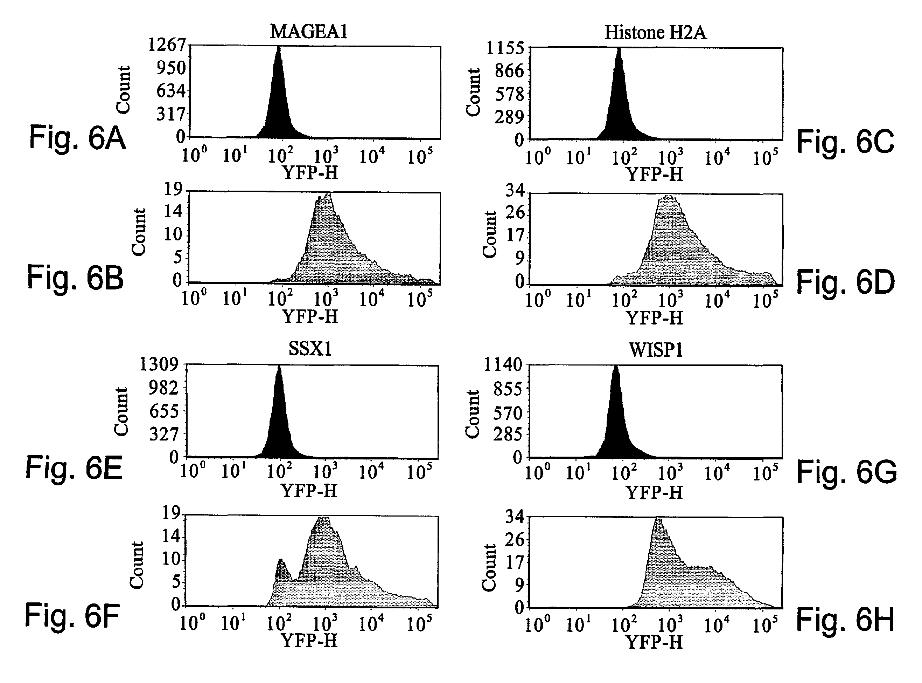

FIGs. 6A-H are representative distributions of YFP positive and YFP negative cells, which were transfected with auxiliary plasmids pPromoter-Au (Figure 4A), in a single population. Following transfection, YFP fluorescence was measured in the LSRII FACS machine. Negative control population (cells with plasmids in which no promoter regulates YFP expression) was used to determine the region which includes YFP negative cells (region P1; Figures 6A, C, E and G). Positive control population (in which YFP expression was regulated by a CMV promoter) was used to determine the region which included YFP positive cells (region P2, Figures 6B, D, F and H). This was preformed using a 2D-graph which shows the distribution of YFP fluorescence (Y-axis) as a function of non-specific fluorescence (X-axis). For each promoter, a histogram of YFP expression distribution in either region P1 (Figures 6A, C, E and G, blue), or region P2 (Figures 6B, D, F and H, red) is shown.
FIG. 7 is a scheme of the retroviral system, based on the Moloney Murine Leukemia Virus (MoMuLV). A retroviral derivative was used in which the promoter/enhancer region of the 3' LTR is deleted (*3'LTRdel*). As a result, expression driven by the 3' LTR is depleted. To examine whether transcription of an upstream element affect the regulation of downstream elements, a retrovirus was constructed in which CFP expression is regulated by an SV40 promoter. Immediately downstream to the CFP (which contained a 3' STOP codon) YFP was cloned.
FIGs. 8A-E are maps of exemplary constructs of the present invention.
FIG. 9 is a bar graph illustrating cancer detection in 293T cells by analysis of the activity of two non-identical promoters. The y-axis shows the output normalized by the expression level in cells transfected with plasmid pOUTPUT only to yield fold-induction.
FIG. 10 is a bar graph illustrating cancer detection in 293T cells by analysis of the activity of two identical promoters. The y-axis shows the output normalized by the expression level in cells transfected with plasmid pOUTPUT only to yield fold-induction.
FIG. 11 is a 3D plot of the output response to the mutual activity of two promoters, for both DocS-Coh2 and MyoD-ID systems: promoters' 1 & 2 activity levels are on the x, y axes; system output levels on z-axis. The x,y axes are in units of YFP fluorescence; z axis is in terms of fold induction Luciferase expression. The plot shows that significant output is generated only when both inputs are high.

### DESCRIPTION OF THE PREFERRED EMBODIMENTS

The present invention, in some embodiments thereof, relates to a nucleic acid construct system which serves as an autonomous molecular computer and, to the use of same for the diagnosis and/or treatment of a cell state.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Many promoters exist which are regulated by a metabolic or a pathological state. Methods for detecting a cell state based on the activity of a particular promoter have been developed for diseases such as cancer. These methods, however, were limited in their implementation, since a single-gene input was shown to be sufficient to discriminate cancer cells from normal ones only in a limited number of tissues and for limited types of cancers [Ohana, P., et al., Int J Cancer, 2002. 98(5): p. 645-50; Fellig, Y., et al., J Clin Pathol, 2005. 58(10): p. 1064-8; Ariel, I., et al., Mol Pathol, 2000. 53(6): p. 320-3].

The present inventors have devised an autonomous system which is capable of integrating at least two gene input signals. Specifically, the present inventors have developed an autonomous system which measures the activity levels of predetermined genes, integrates these signals with a simple computation to generate a biological output once a state of interest is identified. This output can either report the cell's state or intervene with cell-fate.

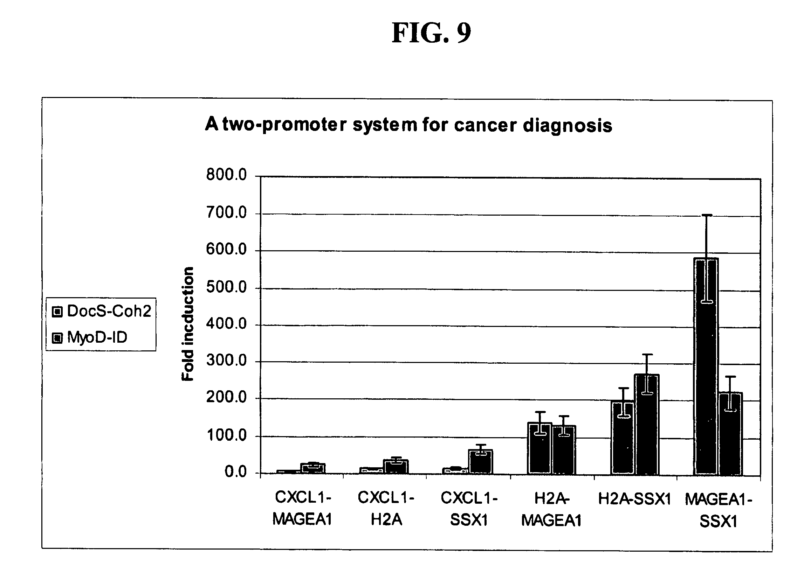

As proof of concept, the present inventors have generated a nucleic acid construct system which generates a reporter polypeptide in response to the concurrent activity of two cancer-related genes in mammalian cells (Figures 9-11).

Thus, according to one aspect of the present invention, there is provided a nucleic acid construct system, comprising:
(i) a first nucleic acid construct comprising a first polynucleotide, the first polynucleotide comprising a first nucleic acid sequence encoding a first expression product, the first nucleic acid sequence being operably linked to a first inducible mammalian transcriptional regulatory sequence; and
(ii) a second nucleic acid construct comprising a second polynucleotide, the second polynucleotide comprising a second nucleic acid sequence encoding a second expression product, the second nucleic acid sequence being operably linked to a second inducible mammalian transcriptional regulatory sequence,
wherein the first mammalian inducible transcriptional regulatory sequence and the second mammalian inducible transcriptional regulatory sequence are regulated by a metabolic state or a pathological state.

The first and second nucleic acid constructs of the present system typically serve as sensor molecules generating independent signals in response to the activity of the inducible transcriptional regulatory sequences comprised therein. The construct system of the present invention may also comprise a third nucleic acid construct which, itself acts as a processor molecule integrating the two signals generated by the first and second nucleic acid constructs. Depending on the nature of the third nucleic acid construct, the system as a whole can be used either to detect a cell state (e.g. disease) or to react to a cell state (e.g. treat a disease).

The term "polynucleotide" as used herein refers to a deoxyribonucleic acid sequence composed of naturally-occurring bases, sugars and covalent internucleoside linkages (e.g., backbone) as well as oligonucleotides having non-naturally-occurring portions which function similarly to respective naturally-occurring portions. Such modifications are enabled by the present invention provided that recombinant expression is still allowed. The polynucleotides may comprise complementary polynucleotide sequences (cDNA), genomic polynucleotide sequences and/or a composite polynucleotide sequences (e.g., a combination of the above).

As used herein the phrase "complementary polynucleotide sequence" refers to a sequence, which results from reverse transcription of messenger RNA using a reverse transcriptase or any other RNA dependent DNA polymerase. Such a sequence can be subsequently amplified *in vivo* or *in vitro* using a DNA dependent DNA polymerase.

As used herein the phrase "genomic polynucleotide sequence" refers to a sequence derived (isolated) from a chromosome and thus it represents a contiguous portion of a chromosome.

As used herein the phrase "composite polynucleotide sequence" refers to a sequence, which is at least partially complementary and at least partially genomic. A composite sequence can include some exonal sequences required to encode the polypeptide of the present invention, as well as some intronic sequences interposing therebetween. The intronic sequences can be of any source, including of other genes, and typically will include conserved splicing signal sequences. Such intronic sequences may further include cis acting expression regulatory elements.

As used herein, the phrase "first expression product" and "second expression product" refer to either polypeptides (including short peptides capable of interfering with the activity of specific polypeptides) or silencing oligonucleotides including siRNAs and antisense polynucleotides. Exemplary expression products are further described herein below.

As used herein the phrase "transcriptional regulatory sequence" refers to a promoter sequence or a sequence upstream (such as an enhancer), wherein activation thereof regulates (increases or decreases) transcription of a nucleic acid sequence operably linked thereto.

The transcriptional regulatory sequences of the constructs of the present invention are regulated by a metabolic state or a pathological state, Accordingly, the transcriptional regulatory sequences of the present invention typically comprise sequences that serve as potential binding sites for transcription factors whose expression or activity are regulated during a particular metabolic or pathological state.

It will be appreciated that a myriad of transcription factors are regulated during pathological and metabolic states and the present invention contemplates all transcriptional regulatory sequences that are capable of binding such transcription factors. Particular examples of disease-regulated transcriptional regulatory sequences include, but are not limited to cancer, neurodegenerative disease, metabolic disease and inflammation transcriptional regulatory sequences. Exemplary metabolic state regulated sequences may include sequences that are regulated by a high or low glucose concentration and sequences that are regulated by a state of proliferation and/or senescence. Exemplary transcriptional sequences that are activated in a cancerous state are described herein below.

According to a particular embodiment of this aspect of the invention, the first and second expression product are able to interact to form a complex capable of regulating (activating or suppressing) expression from the third nucleic acid construct.

The first and second expression product may be any polypeptides of interest, with one limitation: the two must be able to bind with, at least, a minimal affinity via protein-protein interactions.

As used herein, the phrase "minimal affinity" refers to one that is sufficient to drive transcription from the output promoter upon binding of the first and second expression products. Typically, the minimal activity refers to a Kd of at least 10⁻⁶ and more preferably 10⁻⁷.

A method of selecting which expression products to use for a particular construct system has been described by Nissim et al [Phys. Biol. 4 (2007), 154-163].

Exemplary first and second expression product pairs that may be used in the systems of the present invention include, but are not limited to antibody/antigen pairs, lectin/carbohydrate pairs, nucleic acid/nucleic acid pairs, receptor/receptor ligand (e.g. IL-4 receptor and IL-4) pairs, avidin/biotin pairs, etc. Particular examples include wild-type or mutant derivatives of the bacterial DocS and Coh2 as expression product 1 and expression product 2. Mutant [or truncated] derivatives may be used when it is desired to decrease the affinity between the two expression products. Exemplary mutant derivatives of DocS and Coh2 which may be used as expression products are disclosed in Fierobe, H.P., et al., J Biol Chem, 2001. 276(24): p. 21257-61; Handelsman, T., et al., FEBS Lett, 2004. 572(1-3): p. 195-200; Barak, Y., et al., J Mol Recognit, 2005. 18(6): p. 491-501.

For detecting/treating cancer using the embodiment described herein above, the present inventors contemplate detecting an activity pattern of two or more genes whose mutual activity is possible only in the aberrant cells.

One category of transcriptional regulatory sequences that may be used in the constructs of the present invention is cell-cycle regulated transcriptional regulatory sequences.

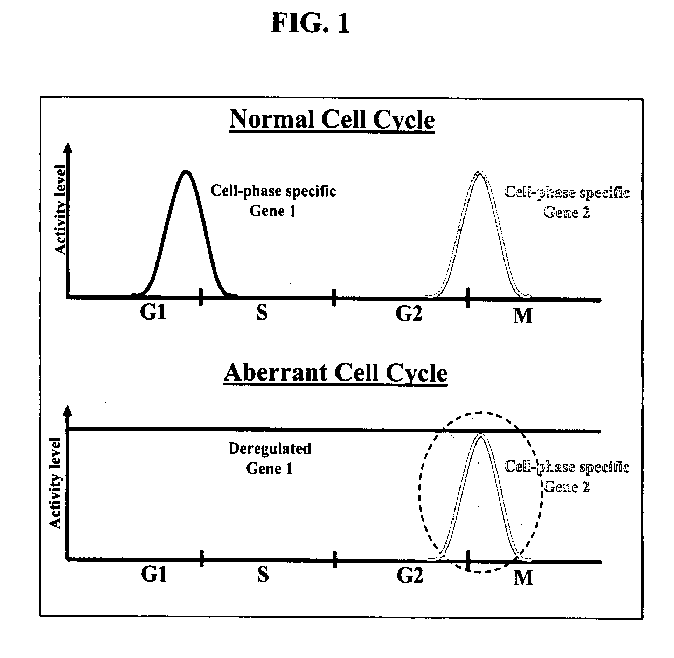

The cell-cycle is a tightly regulated process in which the activity of genes is highly orchestrated in response to both inter-cellular signals, such as growth factors, and to intra-cellular signals, such as the concentrations of essential biochemicals, cell size, DNA replication and DNA-damage. The cell cycle is divided into four sequential phases: G1, S, G2 and M, Each phase is characterized by the expression and activity of phase-specific proteins, such as cyclins, cyclin-dependent kinases (CDK's) and the RB-E2F1 complex. The tight coordination between phase-specific genes activity and cell-phase is extremely important for the regulation and maintenance of normal cell cycle. Therefore, in normal cells, cell cycle phase-specific genes are active only at a specific phase. Deregulation of cell cycle genes can cause aberrant growth and cancer. Therefore, analysis of cell-cycle gene expression patterns can be used to identify malignant transformation. For example, detection of a product of a G1 phase-specific gene and, simultaneously, a product of a G2-M phase-specific gene must be the result of deregulated cell cycle (Figure 1).

Exemplary pairs of cell-cycle transcriptional regulatory sequences that may be used in the first and second constructs of the present invention are listed in Table 1 herein below.

**Table 1**

| ***Tanscriptional regulatory sequence 1*** | ***Tanscriptional regulatory sequence 1*** |
|---|---|
| E2F1 (G1/S) | BCRA1/2 (s) |
| Prb (G1) | CDKN2C (S/G2) |
| CDC25 (G2/M) | Histone H2A/B (S) |
| p21 (G1/S) | CCNA2 (G2) |
| p73 (G1/S) | BUB1 (G2/M) |
| DHFR (S) | CDC25B/C (G2/M) |
| Cyclin E (G1/S) | CDKN2D (G2/M) |
| Cyclin A (S) | CKS1/2 (G2/M) |

Other cell-cycle transcriptional regulatory sequences that are contemplated for use in the first and second constructs of the present invention are taught in Morgan, D.O., Annu Rev Cell Dev Biol, 1997. 13: p. 261-91; Muller, H., et al., Genes Dev, 2001. 15(3): p. 267-85; Whitfield, M.L., et al., Mol Biol Cell, 2002. 13(6): p. 1977-2000; Tsantoulis, P.K. and V.G. Gorgoulis, Eur J Cancer, 2005. 41(16): p. 2403-14.

It will be appreciated that mutual activity of two transcriptional regulatory sequences other than cell-cycle regulated transcriptional regulatory sequences may also be indicative of cancer and these may also be incorporated in the constructs of the present invention. Thus for example Chuang et al, Mol Systems Biol, 16, October 2007, teach that amongst a large number of pairs, simultaneous up-regulation of [E2F1 and RAD51] or [VEGFβ and SHC1] indicate high metastasis potential.

Other exemplary pairs of transcriptional regulatory sequences that may be used in the constructs of the present invention include tissue specific transcriptional regulatory sequences. Two transcriptional regulatory sequences specific for two different tissues can be active individually in normal cells, but can be mutually active only in cancer cells. Thus for example, [CXCL1 and ILIB], [CXCL1 and MMP3] or [MMP3 and IL1B] are active simultaneously in tumor cells, but not in normal cells, as seen in human embryonic lung fibroblast cancer model [Milyavsky, M., et al., Cancer Res, 2005. 65(11): p. 4530-43; Milyavsky, M., et al., Cancer Res, 2003. 63(21): p. 7147-57].

Other optional transcriptional regulatory sequences (and their polynucleotide sequences) and output proteins are listed in Table 2, herein below.

**Table 2**

| ***Transcriptional regulatory sequence 1*** | ***Transcriptional regulatory sequence 1*** | **Protein A** | **Protein B** |
|---|---|---|---|
| CXCL1 (SEQ ID NO: 1) | CXCL1 (SEQ ID NO: 1) | DocS/VP16-AD | Coh2/GAL4-BD |
| CXCL1 (SEQ ID NO: 1) | Histone-H2A (SEQ ID NO: 2) | DocS/VP16-AD | Coh2/GAL4-BD |
| CXCL1 (SEQ ID NO: 1) | MAGEA 1 (SEQ ID NO: 3) | DocS/VP16-AD | Coh2/GAL4-BD |
| CXCL1 (SEQ ID NO: 1) | SSX1 (SEQ ID NO: 5) | DocS/VP16-AD | Coh2/GAL4-BD |
| CXCL1 (SEQ ID NO: 1) | WISP (SEQ ID NO: 6) | DocS/VP16-AD | Coh2/GAL4-BD |
| Histone-H2A (SEQ ID NO: 2) | CXCL1 (SEQ ID NO: 1) | DocS/VP16-AD | Coh2/GAL4-BD |
| Histone-H2A (SEQ ID NO: 2) | Histone-H2A (SEQ ID NO: 2) | DocS/VP16-AD | Coh2/GAL4-BD |
| Histone-H2A (SEQ ID NO: 2) | MAGEA1 (SEQ IDNO:3) | DocS/VP16-AD | Coh2/GAL4-BD |
| Histone-H2A (SEQ ID NO: 2) | SSX1 (SEQ ID NO: 5) | DocS/VP16-AD | Coh2/GAL4-BD |
| Histone-H2A (SEQ ID NO: 2) | WISP1 (SEQ ID NO: 6) | DocS/VP16-AD | Coh2/GAL4-BD |
| MAGEA1 (SEQ ID NO: 3) | CXCL1 (SEQ ID NO: 1) | DocS/VP16-AD | Coh2/GAL4-BD |
| MAGEA1 (SEQ ID NO: 3) | Histone-H2A (SEQ ID NO: 2) | DocS/VP16-AD | Coh2/GAL4-BD |
| MAGEA1 (SEQ ID NO: 3) | MAGEA1 (SEQ ID NO: 3) | DocS/VP16-AD | Coh2/GAL4-BD |
| MAGEA1 (SEQ ID NO: 3) | SSX1 (SEQ ID NO: 5) | DocS/VP16-AD | Coh2/GAL4-BD |
| MAGEA1 (SEQ ID NO: 3) | WISP1 (SEQ ID NO: 6) | DocS/VP16-AD | Coh2/GAL4-BD |
| SSX1 (SEQ ID NO: 5) | CXCL1 (SEQ ID NO: 1) | DocS/VP16-AD | Coh2/GAL4-BD |
| SSX1(SEQ ID NO: 5) | Histone-H2A (SEQ ID NO: 2) | DocS/VP16-AD | Coh2/GAL4-BD |
| SSX1(SEQ ID NO: 5) | MAGEA1 (SEQ ID NO: 3) | DocS/VP16-AD | Coh2/GAL4-BD |
| SSX I (SEQ ID NO: 5) | SSX1(SEQ ID NO: 5) | DocS/VP16-AD | Coh2/GAL4-BD |
| SSX1(SEQ ID NO: 5) | WISP (SEQ ID NO: 6) | DocS/VP16-AD | Coh2/GAL4-BD |
| WISP1 (SEQ ID NO: 6) | CXCL1 (SEQ ID NO: 1) | DocS/VP16-AD | Coh2/GAL4-BD |
| WISP1 (SEQ ID NO: 6) | Histone-H2A (SEQ ID NO: 2) | DocS/VP16-AD | Coh2/GAL4-BD |
| WISP1 (SEQ ID NO: 6) | MAGEA1 (SEQ ID NO: 3) | DocS/VP16-AD | Coh2/GAL4-BD |
| WISP1 (SEQ ID NO: 6) | SSX1 (SEQ ID NO: 5) | DocS/VP16-AD | Coh2/GAL4-BD |
| WISP (SEQ ID NO: 6) | WISP1 (SEQ ID NO: 6) | DocS/VP16-AD | Coh2/GAL4-BD |

It has been shown that down-regulation of tumor suppressors, such as [p21 *AND* p16] is tightly correlated with the activation of the proliferation cluster genes and, consequently, with the proliferation rate of human embryonic lung fibroblast [Tabach Y et al., Mol Syst Biol, 2005. 1: p. 2005 0022]. Thus, as another example, a high activity of two tumor suppressor promoters (e.g. p21 and p 16) may be used to indicate that a cell is not cancerous, whereas a low activity of the tumor suppressor promoters may be used to indicate that a cell is cancerous.

Accordingly, the present invention contemplates other embodiments for the integration of the signals from the first and second constructs of the present invention. Thus for example, tumor suppressor gene transcriptional regulatory elements can be used in the second nucleic acid construct in order to drive the expression of an siRNA molecule, an RNAzyme or an antisense polynucleotide which inhibits the expression of the system output-protein (i.e. the reporter polypeptide) or the signal emitted from the first nucleic acid construct. One possible mechanism can be a three-module system which includes an E2F1 promoter regulating the expression of a transcriptional activator (e.g. GAL4-BD/VP16-AD fusion protein), which itself is capable of inducing the output-gene expression from a GAL4 promoter, a p21 promoter regulating the expression of a siRNA molecule which represses the translation of the output protein and a promoter (e.g. GAL4) which regulate the expression of the output protein. This would constitute a [E2F1 *NOT* p21] gate.

The term "siRNA" as used herein, refers to small interfering RNAs, which also include short hairpin RNA (shRNA) [Paddison et al., Genes & Dev. 16: 948-958, 2002], that are capable of causing interference and can cause post-transcriptional silencing of specific genes in cells, for example, mammalian cells (including human cells) and in the body, for example, mammalian bodies (including humans).

RNA interference is a two step process. The first step, which is termed as the initiation step, input dsRNA is digested into 21-23 nucleotide (nt) small interfering RNAs (siRNA), probably by the action of Dicer, a member of the RNase III family of dsRNA-specific ribonucleases, which processes (cleaves) dsRNA (introduced directly or via a transgene or a virus) in an ATP-dependent manner. Successive cleavage events degrade the RNA to 19-21 bp duplexes (siRNA), each with 2-nucleotide 3' overhangs [Hutvagner and Zamore Curr Opin Genetics and Development 12:225-232 (2002); and Bernstein, Nature 409:363-366 (2001)].

In the effector step, the siRNA duplexes bind to a nuclease complex from the RNA-induced silencing complex (RISC). An ATP-dependent unwinding of the siRNA duplex is required for activation of the RISC. The active RISC then targets the homologous transcript by base pairing interactions and cleaves the mRNA into 12 nucleotide fragments from the 3' terminus of the siRNA [Hutvagner and Zamore Curr Op Gen Develop. 12:225-232 (2002); Hammond et al., 2001. Nat Rev Gen. 2:110-119 (2001); and Sharp Genes Dev. 15:485-90 (2001)]. Although the mechanism of cleavage is still to be elucidated, research indicates that each RISC contains a single siRNA and an RNase [Hutvagner and Zamore, Curr Opin Gen. Develop. 12:225-232 (2002)].

Because of the remarkable potency of RNAi, an amplification step within the RNAi pathway has been suggested. Amplification could occur by copying of the input dsRNAs which would generate more siRNAs, or by replication of the siRNAs formed. Alternatively or additionally, amplification could be effected by multiple turnover events of the RISC [Hammond el al., Nat Rev Gen. 2:110-119 (2001), Sharp Genes Dev. 15:485-90 (2001); Hutvagner and Zamore Curr Opin Gen. Develop. 12:225-232 (2002)]. Ample guidance for using RNAi to practice the present invention is provided in the literature of the art [refer, for example, to: Tuschl, ChemBiochem. 2:239-245 (2001) incorporated herein by reference; Cullen, Nat Immunol. 3:597-599 (2002) incorporated herein by reference; and Brantl, Biochem Biophys Acta 1575:15-25 (2002) incorporated herein by reference].

Synthesis of RNAi molecules suitable for use with the present invention can be effected as follows. First, the mRNA sequence encoding the polypeptide of the present invention is scanned downstream of the AUG start codon for AA dinucleotide sequences. Occurrence of each AA and the 3' adjacent 19 nucleotides is recorded as potential siRNA target sites. Preferably, siRNA target sites are selected from the open reading frame, as untranslated regions (UTRs), being enriched in regulatory protein binding sites. UTR-binding proteins and/or translation initiation complexes may interfere with binding of the siRNA endonuclease complex [Tuschl, Chem Biochem. 2:239-245]. It will be appreciated though, that siRNAs directed at untranslated regions may also be effective, as demonstrated for GAPDH wherein siRNA directed at the 5' UTR mediated approximately 90 % decrease in cellular GAPDH mRNA and completely abolished protein level www.ambion.com/techlib/tn/142/3.html or www.ambion.com/techlib/tnll3l/4.html.

Second, potential target sites are compared to an appropriate genomic database (e.g., human, mouse, rat etc.) using any sequence alignment software, such as the BLAST software available from the NCBI server (www.ncbi.nlm.nih.gov/BLAST/). Putative target sites which exhibit significant homology to other coding sequences are filtered out.

Qualifying target sequences are selected as template for siRNA synthesis. Preferred sequences are those including low G/C content as these have proven to be more effective in mediating gene silencing as compared to those with G/C content higher than 55 %. Several target sites are preferably selected along the length of the target gene for evaluation. For better evaluation of the selected siRNAs, a negative target gene for evaluation. For better evaluation of the selected siRNAs, a negative control is preferably used in conjunction. Negative control siRNA preferably include the same nucleotide composition as the siRNAs but lack significant homology to the genome. Thus, a scrambled nucleotide sequence of the siRNA is preferably used, provided it does not display any significant homology to any other gene.

Another agent capable of inhibiting the expression of the system output-protein or the signal emited from the first nucleic acid is an antisense polynucleotide capable of specifically hybridizing with an mRNA transcript.

Design of antisense molecules which can be used to efficiently downregulate the system output protein must be effected while considering two aspects important to the antisense approach. The first aspect is delivery of the oligonucleotide into the cytoplasm of the appropriate cells, while the second aspect is design of an oligonucleotide which specifically binds the designated mRNA within cells in a way which inhibits translation thereof.

The prior art teaches of a number of delivery strategies which can be used to efficiently deliver oligonucleotides into a wide variety of cell types [see, for example, Luft J Mol Med 76: 75-6 (1998); Kronenwett et al. Blood 91: 852-62 (1998); Rajur et al. Bioconjug Chem 8: 935-40 (1997); Lavigne et al. Biochem Biophys Res Commun 237: 566-71 (1997) and Aoki et al. (1997) Biochem Biophys Res Commun 231: 540-5 (1997)].

In addition, algorithms for identifying those sequences with the highest predicted binding affinity for their target mRNA based on a thermodynamic cycle that accounts for the energetics of structural alterations in both the target mRNA and the oligonucleotide are also available [see, for example, Walton et al. Biotechnol Bioeng 65: 1-9 (1999)].

Such algorithms have been successfully used to implement an antisense approach in cells. For example, the algorithm developed by Walton et al. enabled scientists to successfully design antisense oligonucleotides for rabbit beta-globin (RBG) and mouse tumor necrosis factor-alpha (TNF alpha) transcripts. The same research group has more recently reported that the antisense activity of rationally selected oligonucleotides against three model target mRNAs (human lactate dehydrogenase A and B and rat gp130) in cell culture as evaluated by a kinetic PCR technique proved effective in almost all cases, including tests against three different targets in two cell types with phosphodiester and phosphorothioate oligonucleotide chemistries.

In addition, several approaches for designing and predicting efficiency of specific oligonucleotides using an in vitro system were also published (Matveeva et al., Nature Biotechnology 16: 1374 - 1375 (1998)].

The current consensus is that recent developments in the field of antisense technology which, as described above, have led to the generation of highly accurate antisense design algorithms and a wide variety of oligonucleotide delivery systems, enable an ordinarily skilled artisan to design and implement antisense approaches suitable for downregulating expression of known sequences without having to resort to undue trial and error experimentation.

Another agent capable of downregulating the system output-protein or the signal emited from the first nucleic acid is a ribozyme molecule. Ribozymes are being increasingly used for the sequence-specific inhibition of gene expression by the cleavage of mRNAs encoding proteins of interest [Welch et al., Curr Opin Biotechnol. 9:486-96 (1998)]. The possibility of designing ribozymes to cleave any specific target RNA has rendered them valuable tools in both basic research and therapeutic applications. In the therapeutics area, ribozymes have been exploited to target viral RNAs in infectious diseases, dominant oncogenes in cancers and specific somatic mutations in genetic disorders [Welch et al., Clin Diagn Virol. 10:163-71 (1998)]. Most notably, several ribozyme gene therapy protocols for HIV patients are already in Phase 1 trials. More recently, ribozymes have been used for transgenic animal research, gene target validation and pathway elucidation. Several ribozymes are in various stages of clinical trials. ANGIOZYME was the first chemically synthesized ribozyme to be studied in human clinical trials. ANGIOZYME specifically inhibits formation of the VEGF-r (Vascular Endothelial Growth Factor receptor), a key component in the angiogenesis pathway. Ribozyme Pharmaceuticals, Inc., as well as other firms have demonstrated the importance of anti-angiogenesis therapeutics in animal models. HEPTAZYME, a ribozyme designed to selectively destroy Hepatitis C Virus (HCV) RNA, was found effective in decreasing Hepatitis C viral RNA in cell culture assays (Ribozyme Pharmaceuticals, Incorporated - WEB home page).

Another agent capable of downregulating the system output-protein or the signal emitted from the first nucleic acid is a short peptide e.g. genetic suppressor element (GSE) peptide. This mechanism is detailed in Ossovskaya et al [PNAS, Vol 93, p.10309-10314], incorporated herein by reference.

It will be appreciated that the nucleic acid constructs of the present invention may also comprise other elements of regulatory machinery (e.g. initiation site, stop site, degradation tags etc.) sufficient to direct and/or regulate expression of the polynucleotides comprised therein.

Degradation tags (sometimes referred to as PEST sequences) can be used to couple the concentrations of the synthetic chimera proteins (i.e. the first and second expression product and/or the output protein) to the concentrations of a native protein. Since the chimera proteins are heterologous to mammalian cells, they are not subjected to active degradation protein-specific degradation (for example, via the ubiquitin pathway) and therefore have a relatively long half life. Native proteins often carry a degradation tag that targets them for degradation by the cell machinery at a specific cell state. Addition of the degradation tag of the native cell cycle-specific protein to the synthetic protein, will allow it to be degraded in coordination with the native protein. This may give better correlation between the native and synthetic protein.

The degradation tag may be a general degradation tag that shortens the half life of the synthetic protein and might assist in achieving better resolution for cell state identification by preventing the accumulation of the synthetic proteins (for example, as a result of a minor leak from the promoters which regulate the expression of the synthetic proteins).

A possible degradation tag is amino acids 422-461 of the mouse ornithine decarboxylase (MODC). This peptide comprises a PEST sequence which targets the protein to ubiquitination and, subsequently, to degradation. For example, YFP protein fused to this PEST sequence has a half-life of approximately 1-1.5 hours [Li, X., et al., J Biol Chem, 1998. 273(52): p. 34970-5]. A polynucleotide sequence of an exemplary degradation signal is set forth in SEQ ID NO: 15. Other degradation tags could be phase-specific. Such tags would target the fusion proteins to degradation at a specific cell-phase. For example, the 100 N-terminal amino acids of cyclinB1, which targets it for degradation at the G2-M phase [King, R.W., et al., Science, 1996. 274 (5293): p. 1652-9]. Thus, when a fusion protein is regulated by a normal phase-specific promoter and a corresponding phase-specific degradation tag, it would be coupled to the specific cell-phase. Therefore, it should be possible to construct a system in which the two fusion proteins are correlated with two different cell cycle phases. In such a system, the fusion proteins will be co-expressed only when the regulation of at least one phase-specific promoter is disrupted. As a result, the output protein would be expressed only in cells with impaired cell cycle regulation.

The degradation tag may also be an mRNA decay tag, such as described in Wilusz and Wilusz [Trends in Genetics, Vol 20., No. 10, 2004], incorporated herein by reference.

The nucleic acid construct may also comprise a TATA box and other upstream promoter elements. The TATA box, located 25-30 base pairs upstream of the transcription initiation site, is thought to be involved in directing RNA polymerase to begin RNA synthesis. The other upstream promoter elements determine the rate at which transcription is initiated.

Enhancer elements can stimulate transcription up to 1,000 fold from linked homologous or heterologous promoters. Enhancers are active when placed downstream or upstream from the transcription initiation site. Many enhancer elements derived from viruses have a broad host range and are active in a variety of tissues. For example, the SV40 early gene enhancer is suitable for many cell types. Other enhancer/promoter combinations that are suitable for the present invention include those derived from polyoma virus, human or murine cytomegalovirus (CMV), the long term repeat from various retroviruses such as murine leukemia virus, murine or Rous sarcoma virus and HIV. See, Enhancers and Eukaryotic Expression, Cold Spring Harbor Press, Cold Spring Harbor, N.Y. 1983, which is incorporated herein by reference.

In the construction of the nucleic acid construct, the promoter is preferably positioned approximately the same distance from the heterologous transcription start site as it is from the transcription start site in its natural setting. As is known in the art, however, some variation in this distance can be accommodated without loss of promoter function.

Polyadenylation sequences can also be added to the expression vector in order to increase the efficiency of translation of the expression products. Two distinct sequence elements are required for accurate and efficient polyadenylation: GU or U rich sequences located downstream from the polyadenylation site and a highly conserved sequence of six nucleotides, AAUAAA, located 11-30 nucleotides upstream. Termination and polyadenylation signals that are suitable for the present invention include those derived from SV40.

In addition to the elements already described, the nucleic acid constructs of the present invention may typically contain other specialized elements intended to increase the level of expression of cloned nucleic acids or to facilitate the identification of cells that carry the recombinant DNA. For example, a number of animal viruses contain DNA sequences that promote the extra chromosomal replication of the viral genome in permissive cell types. Plasmids bearing these viral replicons are replicated episomally as long as the appropriate factors are provided by genes either carried on the plasmid or with the genome of the host cell.

The vector may or may not include a eukaryotic replicon. If a eukaryotic replicon is present, then the vector is amplifiable in eukaryotic cells using the appropriate selectable marker. If the vector does not comprise a eukaryotic replicon, no episomal amplification is possible. Instead, the recombinant DNA integrates into the genome of the engineered cell, where the promoter directs expression of the desired nucleic acid.

The nucleic acid constructs of the present invention can further include additional polynucleotide sequences that allow, for example, the translation of several proteins from a single mRNA such as an internal ribosome entry site (IRES) and sequences for genomic integration of the promoter-chimeric polypeptide.

According to a further embodiment the first expression product and the second expression product are encoded on a single nucleic acid construct. Furthermore, all three elements (i.e. the first and second expression product and the output protein) may be encoded on a single nucleic acid construct. Typically such constructs would comprise an internal ribosome entry site (IRES).

The nucleic acid constructs of this invention may be prepared by any suitable method, including, for example, cloning and restriction of appropriate sequences or direct chemical synthesis by methods such as the phosphotriester method of Narang et al. (1979) Meth. Enzymol. 68: 90-99; the phosphodiester method of Brown et al. (1979) Meth. Enzymol. 68: 109-151, the diethylphosphoramidite method of Beaucage et al. (1981) Tetra. Lett., 22: 1859-1862; and the solid support method of U.S. Pat. No. 4,458,066.

The first and second nucleic acid constructs may be provided as a kit either alone or in combination with the third nucleic acid construct. The kit may optionally include any reagents and/or apparatus to facilitate practice of the methods described herein below. Such reagents include, but are not limited to buffers, instrumentation (e.g. bandpass filter), reagents for detecting a signal from a reporter gene, transfection reagents, cell lines, vectors, and the like. In addition, the kits may include instructional materials containing directions (i.e., protocols) for the practice of the methods of this invention.

As mentioned, the nucleic acid construct system of the present invention may be used to diagnose a cell state.

Thus, according to another aspect of this invention, there is provided a method of diagnosing a disease or a metabolic state, the method comprising expressing the nucleic acid construct system of the present invention in at least one mammalian cell, wherein a change in expression of the reporter polypeptide is indicative of the disease or metabolic state.

As used herein, the term "diagnosing" refers to determining the presence of a disease, classifying a disease, determining a severity of the disease (grade or stage), monitoring disease progression, forecasting an outcome of the disease and/or prospects of recovery.

Any mammalian cell (e.g. human) can harbor the construct systems of this invention. The cells can include cells in long term culture (e.g. HeLa cells, CHO cells, SW480 cells, SW48 cells, DLD-1, HCT-116, HT29, 293 cells, U-20S, T-47D, MCF-7, A549, Hep G, Jarkat cells, and the like. The cells can also include acute (unpassaged) cells and cells in vivo.

Diseases that may be diagnosed according to this aspect of the present invention are typically multi-faceted diseases that are not dependent on a genetic defect of a single gene. Exemplary diseases include proliferative disorders such as cancer, metabolic disorders such as diabetes, neurodegenerative disorders, inflammation-associated disorders and immune associated disorders.

According to this aspect of the present invention, the third nucleic acid construct comprises a promoter operably linked to a reporter polypeptide.

A "reporter polypeptide" as used herein, refers to a polypeptide whose expression indicates the simultaneous expression and/or level of expression of the two input signals. According to one embodiment the reporter polypeptide comprises a detectable moiety. Polypeptides comprising detectable moieties are well known to those of skill in the art. They include, but are not limited to, bacterial chloramphenicol acetyl transferase (CAT), beta-galactosidase, green fluorescent protein (GFP) and other fluorescent protein, various bacterial luciferase genes, e.g., the luciferase genes encoded by Vibrio harveyi, Vibrio fischeri, and Xenorhabdus luminescens, the firefly luciferase gene FFlux, and the like.

It will be appreciated that reporter polypeptides may also be detected even in the absence of a "traditional" detectable moiety, such as those listed above. Generally the transcription, translation, or activity of any gene can routinely be detected. Thus, for example, a reporter may be detected by methods including, but not limited to, Northern blots, amplification techniques (e.g. PCR), and the like. Similarly, the translated protein product can be detected by detecting the characteristic activity of the protein or by detecting the protein product itself (e.g. via Western blot, capillary electrophoresis, and the like). It will be appreciated that the reporter polypeptide may be a secreted polypeptide. Accordingly, the present invention contemplates detecting the reporter in a biological fluid, such as blood or urine.

The method according to this aspect of the present invention is typically effected by expressing the constructs of the present invention in a test cell wherein a change (i.e. an up-regulation or down-regulation) of the reporter polypeptide is indicative of the disease. Non-diseased cells, preferably identical to the test cells (e.g. identical cell type, derived from a subject of the same sex, age, weight etc.), may be transfected/infected with the expression constructs of the present invention to serve as controls.

As mentioned, the nucleic acid construct system of the present invention may also be used to treat a disease.

Thus, according to another aspect of the present invention, there is provided a method of treating a disease, the method comprising expressing the nucleic acid construct system of the present invention, in at least one cell of a subject in need thereof, thereby treating the disease.

As used herein the term "subject in need thereof" refers to a mammal, preferably a human subject.

As used herein the term "treating" refers to preventing, curing, reversing, attenuating, alleviating, minimizing, suppressing or halting the deleterious effects of a disease or condition.

According to this aspect of the present invention, the system output protein (i.e. the reporter polypeptide) of the third nucleic acid construct comprises a therapeutic polypeptide. The therapeutic polypeptide may encode an agent that can be used to selectively kill or inhibit a cell that expresses the expression products from the first and second nucleic acid construct. Alternatively, the system output polypeptide may be active in all states besides the state of interest. For example, the system output polypeptide may be the product of a helper gene that would rescue all cells but the diseased ones.

According to one embodiment, the therapeutic polypeptide is a cytotoxic polypeptide. Such therapeutic molecules may be useful for killing cancerous cells for example.

As used herein, the phrase "a cytotoxic polypeptide" refers to a polypeptide that when expressed results in cell death or renders the cell susceptible to killing by another reagent. Thus, for example, expression of a herpes virus thymidine kinase gene will render a cell susceptible to the drug gangcyclovir which will cause the selective killing of any cell producing it. Suitable cytotoxic polypeptides include, but are not limited to Psuedomonas exotoxin, Diphtheria toxin, ricin, abrin, thymidine kinase (e.g. TK1), apoptosis genes, and genes involved in an apoptosis related pathway (e.g. P53, P73, Bax, Bad, FADD, caspases, etc.).

It will be appreciated that therapeutic polypeptides other than cytotoxic polypeptides are also contemplated by the present invention. Selection of a particular therapeutic polypeptide is dependent on the disease which is being treated.

The nucleic acid constructs can be transferred into the chosen host cell ex-vivo by well-known methods such as by electroporation for mammalian cells. In vivo transfection can be accomplished using standard gene therapy methods, e.g. as described herein. In addition, the cells may be transfected with the nucleic acid constructs of the present invention directly (e.g. via microinjection, lipid encapsulation, HIV TAT protein mediated transfer, etc.). In particular, it is noted that the human immunodeficiency virus TAT protein (HIV TAT), when fused to considerably larger proteins results in delivery of the biologically active protein even across the blood brain barrier (see, e.g., Schwarze et al. (1999) Science, 285: 1569-1572, and references cited therein).

"Gene therapy" as used herein refers to the transfer of genetic material (e.g. DNA or RNA) of interest into a host to treat or prevent a genetic or acquired disease or condition or phenotype. The genetic material of interest encodes a product (e.g. a protein, polypeptide, peptide, functional RNA, antisense) whose production *in vivo* is desired. For example, the genetic material of interest can encode a hormone, receptor, enzyme, polypeptide or peptide of therapeutic value. For review see, in general, the text "Gene Therapy" (Advanced in Pharmacology 40, Academic Press, 1997).

Two basic approaches to gene therapy have evolved: (1) ex vivo and (2) *in vivo* gene therapy. In ex vivo gene therapy cells are removed from a patient, and while being cultured are treated *in vitro.* Generally, a functional replacement gene is introduced into the cell via an appropriate gene delivery vehicle/method (transfection, transduction, homologous recombination, etc.) and an expression system as needed and then the modified cells are expanded in culture and returned to the host/patient. These genetically reimplanted cells have been shown to express the transfected genetic material in situ. The cells may be autologous or non-autologous to the subject. Since non-autologous cells are likely to induce an immune reaction when administered to the body several approaches have been developed to reduce the likelihood of rejection of non-autologous cells. These include either suppressing the recipient immune system or encapsulating the non-autologous cells in immunoisolating, semipermeable membranes before transplantation.

In *in vivo* gene therapy, target cells are not removed from the subject rather the genetic material to be transferred is introduced into the cells of the recipient organism in situ, that is within the recipient. In an alternative embodiment, if the host gene is defective, the gene is repaired in situ (Culver, 1998. (Abstract) Antisense DNA & RNA based therapeutics, February 1998, Coronado, CA).

Introduction of nucleic acids by infection in both in vivo and ex vivo gene therapy offers several advantages over the other listed methods. Higher efficiency can be obtained due to their infectious nature. Moreover, viruses are very specialized and typically infect and propagate in specific cell types. Thus, their natural specificity can be used to target the vectors to specific cell types *in vivo* or within a tissue or mixed culture of cells. Viral vectors can also be modified with specific receptors or ligands to alter target specificity through receptor mediated events.

In addition, recombinant viral vectors are useful for *in vivo* expression of a desired nucleic acid because they offer advantages such as lateral infection and targeting specificity. Lateral infection is inherent in the life cycle of, for example, retrovirus and is the process by which a single infected cell produces many progeny virions that bud off and infect neighboring cells. The result is that a large area becomes rapidly infected, most of which was not initially infected by the original viral particles. This is in contrast to vertical-type of infection in which the infectious agent spreads only through daughter progeny. Viral vectors can also be produced that are unable to spread laterally. This characteristic can be useful if the desired purpose is to introduce a specified gene into only a localized number of targeted cells.

Typically, viruses infect and propagate in specific cell types. The targeting specificity of viral utilizes its natural specificity of viral vectors utilizes its natural specificity to specifically target predetermined cell types and thereby introduce a recombinant gene into the infected cell. The vector to be used in the methods of the invention will depend on desired cell type to be targeted and will be known to those skilled in the art.

Exemplary viruses that may be used to introduce the nucleic acid constructs of the present system into a cell include adenoviruses and retroviruses such as lentiviruses. Methods of synthesizing retroviral constructs are described in Example 5 herein below.

The constructs of the present invention can be provided to the individual *per se*, or as part of a pharmaceutical composition where it is mixed with a pharmaceutically acceptable carrier.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the constructs which are accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases. One of the ingredients included in the pharmaceutically acceptable carrier can be for example polyethylene glycol (PEG), a biocompatible polymer with a wide range of solubility in both organic and aqueous media (Mutter et al. (1979).

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, transmucosal, transnasal, intestinal or parenteral delivery, including intramuscular, subcutaneous and intramedullary injections as well as intrathecal, direct intraventricular, intravenous, inrtaperitoneal, intranasal, or intraocular injections.

Alternately, one may administer the preparation in a local rather than systemic manner, for example, via injection of the preparation directly into a specific region of a patient's body.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the invention may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The preparations described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The preparation of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. More specifically, a therapeutically effective amount means an amount of active ingredients effective to prevent, alleviate or ameliorate symptoms of disease or prolong the survival of the subject being treated.

Determination of a therapeutically effective amount is well within the capability of those skilled in the art.

For any preparation used in the methods of the invention, the therapeutically effective amount or dose can be estimated initially from in vitro assays. For example, a dose can be formulated in animal models and such information can be used to more accurately determine useful doses in humans.

Toxicity and therapeutic efficacy of the active ingredients described herein can be determined by standard pharmaceutical procedures *in vitro,* in cell cultures or experimental animals. The data obtained from these *in vitro* and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. [See e.g., Fingl, et al., (1975) "The Pharmacological Basis of Therapeutics", Ch. 1 p.1].

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

It will be appreciated that the constructs of the present invention can be provided to the individual with additional active agents to achieve an improved therapeutic effect as compared to treatment with each agent by itself. In such therapy, measures (e.g., dosing and selection of the complementary agent) are taken to adverse side effects which may be associated with combination therapies.

Compositions including the preparation of the present invention formulated in a compatible pharmaceutical carrier may also be prepared, placed in an appropriate container, and labeled for treatment of an indicated condition.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert.

The constructs of the present invention may also be used to screen for potential therapeutic agents that are capable of reversing a disease cell phenotype.

Thus, according to another aspect of the present invention, there is provided a method of identifying an agent capable of reversing a disease phenotype of a mammalian cell, the method comprising,
(a) expressing the nucleic acid construct system of the present invention in the mammalian cell, the reporter polypeptide of the third construct comprising a detectable moiety;
(b) contacting the mammalian cell with the agent;
(c) measuring a level of detectable moiety following (b) and optionally prior to (b), wherein a reversion of phenotype is indicative of an agent capable of reversing a diseased phenotype of a mammalian cell.

As used herein, the term "agent" refers to a test composition comprising a biological agent or a chemical agent.

Examples of biological agents that may be tested as potential anti cancer agents according to the method of the present invention include, but are not limited to, nucleic acids, e.g., polynucleotides, ribozymes, siRNA and antisense molecules (including without limitation RNA, DNA, RNA/DNA hybrids, peptide nucleic acids, and polynucleotide analogs having altered backbone and/or bass structures or other chemical modifications); proteins, polypeptides (e.g. peptides), carbohydrates, lipids and "small molecule" drug candidates. "Small molecules" can be, for example, naturally occurring compounds (e.g., compounds derived from plant extracts, microbial broths, and the like) or synthetic organic or organometallic compounds having molecular weights of less than about 10,000 daltons, preferably less than about 5,000 daltons, and most preferably less than about 1,500 daltons.

The candidate agents are preferably contacted with the cells (in vitro, ex vivo or in vivo) for a period long enough to have a therapeutic effect.

According to an embodiment of this aspect of the present invention, the detectable moiety is also assayed prior to contact with the agent so that a comparison may be made prior to and following treatment. Alternatively or additionally control experiments may be effected on other (preferably identical) cells wherein the test agent is contacted at a lower concentration or is absent completely. A difference in expression of the detectable moiety in the presence of the test agent(s) is compared to the expression of the detectable moiety where the test agent is present at a lower concentration or absent indicates that the test agent has an activity on the pathway being assayed. Other embodiments, may utilize a positive control comprising a cell which has been contacted with a known therapeutic agent or, more preferably, a reference agent at a particular concentration. The effect of the test agent is then measured relative to the particular concentration of test agent or reference agent.

It is expected that during the life of a patent maturing from this application many relevant promoters/expression products will be discovered and the scope of the terms "transcriptional regulatory sequence" and "expression product" are intended to include all such new sequences and polypeptides.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorpotaed by reference as if fully set forth herein. Other general references are provided throughout this document. The procedures therein are believed to be well known in the art and are provided for the convenience of the reader. All the information contained therein is incorporated herein by reference.

### EXAMPLE 1

### Calibration of promoter activity level

### MATERIALS AND METHODS

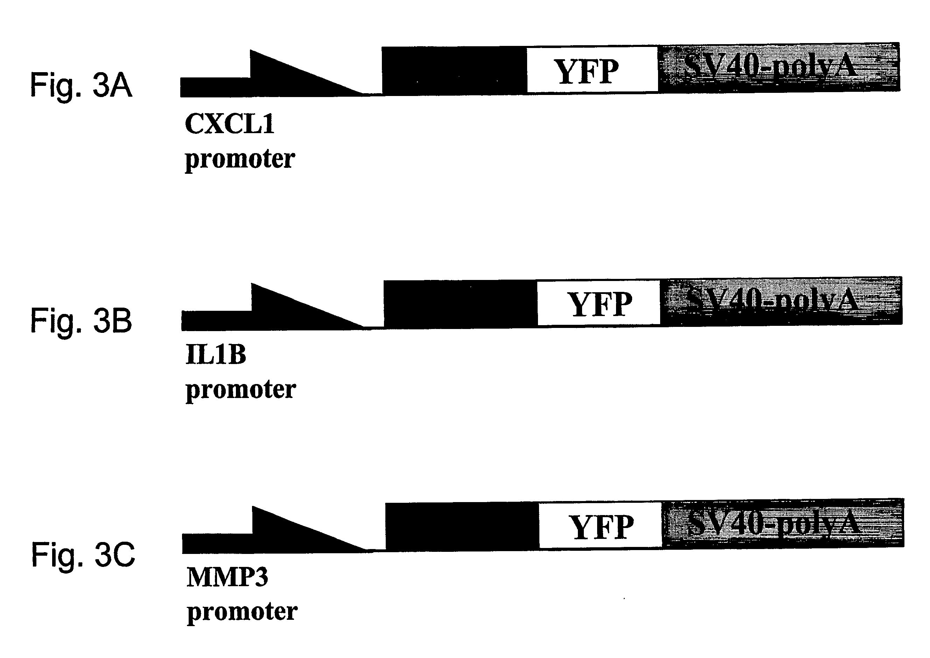

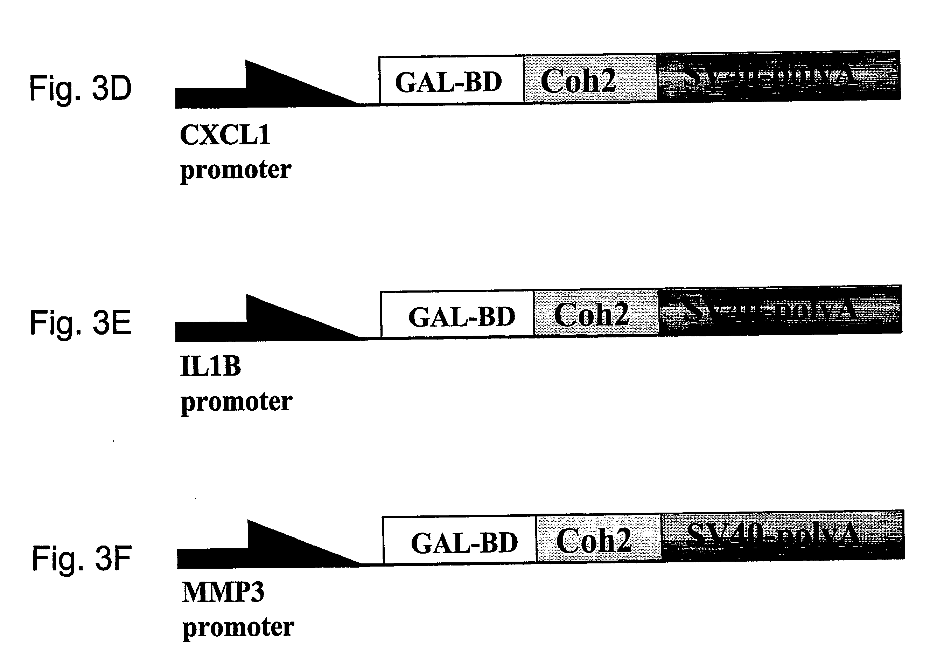

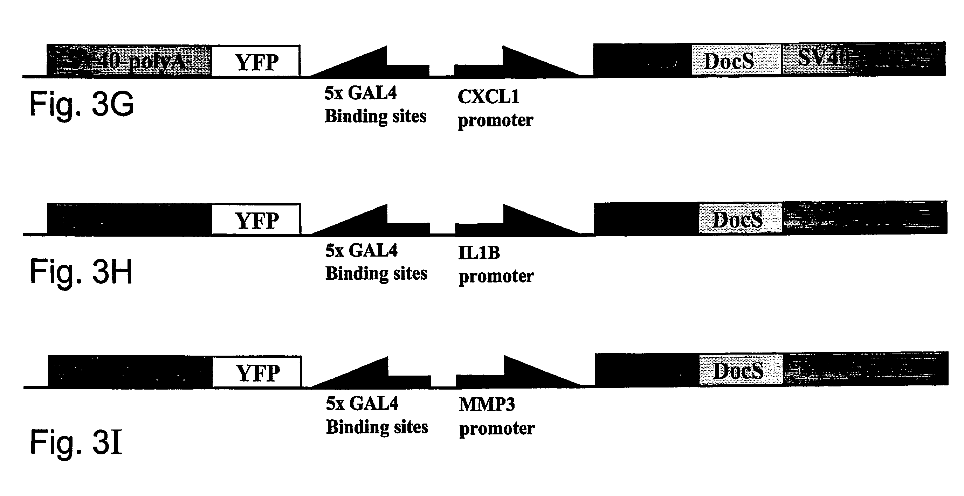

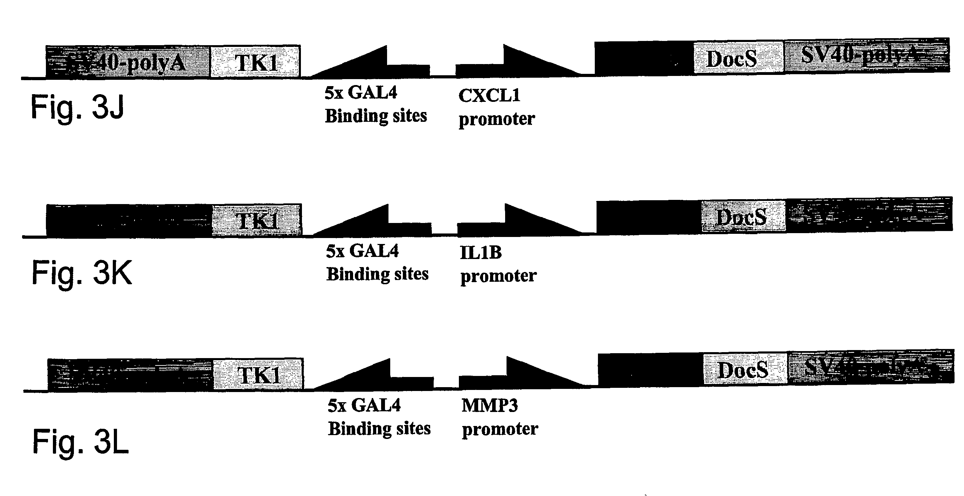

Exemplary constructs that may be used for calibration of promoter activity are illustrated in Figures 3A-C. The construct illustrated in Figure 3A may be used to calibrate CXCL1 promoter activity level (YFP output). The construct illustrated in Figure 3B may be used to calibrate IL1B promoter activity level (YFP output). The construct illustrated in Figure 3C may be used to calibrate MMP3 promoter activity level (YFP output).

WI38 cells, either T\R\G (cancerous) or T\fast (pre-cancerous) [Milyavsky, M., et al., Cancer Res, 2003. 63(21): p. 7147-57; Milyavsky, M., et al., Cancer Res, 2005. 65(11): p. 4530-43], may be used as a model for non-cancer and cancer cells.

### EXAMPLE 2

### Exemplary construct systems for detecting cancer cells

The constructs illustrated in Figures 3D and 3H may be used to detect mutual activity of CXCL 1 (SEQ ID NO: 1) and IL1B (SEQ ID NO: 8) (YFP output; (SEQ ID NO: 9)). The constructs illustrated in Figures 3D and 3I may be used to detect mutual activity of CXCL1 (SEQ ID NO: 1) and MMP3 (SEQ ID NO: 7) (YFP output; (SEQ ID NO: 9)). The constructs illustrated in Figures 3E and 3I may be used to detect mutual activity of IL1B (SEQ ID NO: 8) and MMP3 (SEQ ID NO: 7) (YFP output, (SEQ ID NO: 9)).

WI38 cells, either T\R\G (cancerous) or T\fast (pre-cancerous) [Milyavsky, M., et al., Cancer Res, 2003. 63(21): p. 7147-57; Milyavsky, M., et al., Cancer Res, 2005. 65(11): p. 4530-43], may be used as a model for non-cancer and cancer cells.

### EXAMPLE 3

### Exemplary construct systems for killing cancer cells

The constructs illustrated in Figures 3D and 3K may be used to kill cells with mutual activity of CXCL1 (SEQ ID NO: 1) and IL1B (SEQ ID NO: 8) (TK1 output; (SEQ ID NO: 10)). The constructs illustrated in Figures 3D and 3L may be used to kill cells with mutual activity of CXCL1 (SEQ ID NO: 1) and MMP3 (SEQ ID NO: 7) (TK1 output; (SEQ ID NO: 10)) only in the presence of nucleotide analogues such as BVDU. The constructs illustrated in Figures 3E and 3L may be used to kill cells with mutual activity of IL1B and MMP3 (TK1 output) only in the presence of nucleotide analogues such as BVDU.

W138 cells, either T\R\G (cancerous) or T\fast (pre-cancerous) [Milyavsky, M., et al., Cancer Res, 2003. 63(21): p. 7147-57; Milyavsky, M., et al., Cancer Res, 2005. 65(11): p. 4530-43], may be used as a model for non-cancer and cancer cells.

### EXAMPLE 4

### Plasmid delivery of the constructs of the present invention

For direct transfection, two plasmids were constructed which together comprise the three modules of the network. For modularity, each of the variable components in the system was enclosed in a cassette flanked by two unique restriction sites (plasmids pPromoter-Al, pPromoter-A2 and pPromoter-B, Figures 4B-D). It was, therefore, relatively simple to replace any of the regulating promoters, fusion proteins, or the output protein. The only exception in this case is the PEST sequence. To generate a protein which comprises the PEST peptide, it must be cloned in-frame to the PEST sequence located immediately downstream to *protein-1* and *protein-2*. To exclude the PEST peptide, the protein is cloned with a STOP codon upstream to the PEST sequence. To obtain such modularity, the present inventors had to build these plasmids from a backbone which included only an ampicillin resistance gene and a bacterial ORI (this required an eleven-steps cloning procedure).

Eleven human promoters were selected as candidates for cancer diagnosis. To calibrate the activity of these promoters, approximately 1000 base pairs upstream to the ATG codon (including the 5' UTR) of each gene were cloned upstream to YFP in auxiliary plasmids (plasmid pPromoter-Au, Figure 4A). Plasmids were transfected to the cells using FuGENE-HD transfection reagent (Roche, cat# 04709705001), and YFP measurements were preformed with the LSRII FACS machine.

### RESULTS

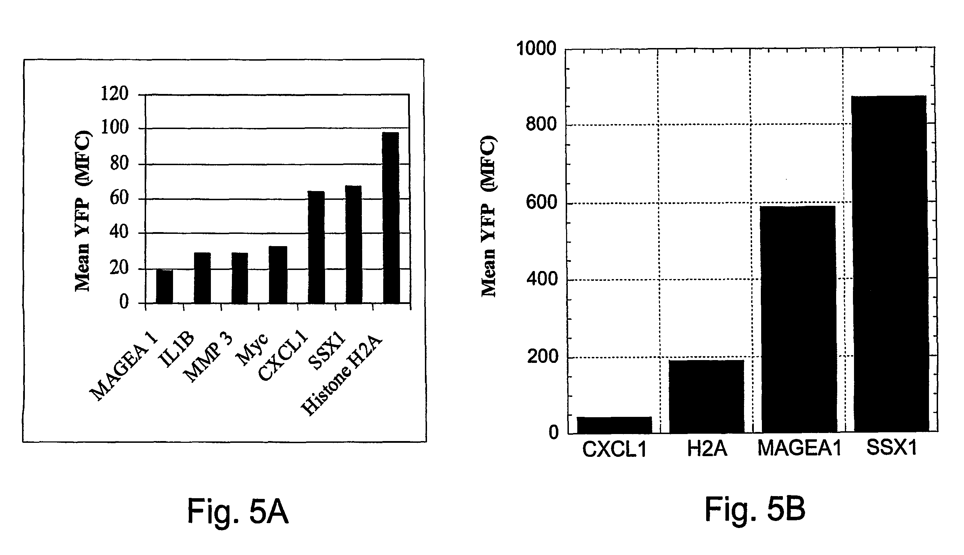

Calibration of promoter activity in 293T and WI38\T\R\G cell lines is shown in Figure 5A-B

Five of these promoters were selected for further study. These include the inflammatory-chemokine CXCL1 promoter [Belperio, J.A., et al., J Clin Invest, 2002. 110(11): p. 1703-16; Wang, D., et al., J Exp Med, 2006. 203(4): p. 941-51], the chromatin structural protein Histone-H2A promoter [Rogakou, E.P., et al., J Biol Chem, 1998. 273(10): p. 5858-68], the Melanoma-associated antigen MAGEAI promoter [De Plaen, E., et al., 1994. 40(5): p. 360-9], the synovial sarcoma X-breakpoint protein-1 (SSX1) promoter [Gure, A.O., et al., Int J Cancer, 1997. 72(6): p. 965-71] and the WNT inducible signaling-pathway protein-1 (WISP1) promoter [Cervello, M., et al., Ann N Y Acad Sci, 2004. 1028: p. 432-9].

Representative distributions of YFP positive and YFP negative cells in a single population following transfection are shown in Figures 6A-H. CXCL1 promoter activity was undetectable in 293T cells, and this promoter will therefore be used as a negative control. All five promoters were cloned to a fully functional network, with wild-type VP16-AD/DocS and GAL4-BD/Coh2 fusion proteins and an YFP output (corresponding plasmids pPromoter-A2 and pPromoter-B, Figures 4C-D).

### EXAMPLE 5

### Retroviral delivery of the constructs of the present invention

For delivery by retroviral infections the Phoenix-Eco system, based on the Moloney Murine Leukemia Virus (MoMuLV) was selected [Nolan, G.P. and A.R. Shatzman, Curr Opin Biotechnol, 1998. 9(5): p. 447-50]. In most retroviral systems, both the 5' LTR and the 3' LTR operate as potent promoters. To ensure that fusion-proteins expression would be regulated only by the promoters of the present invention, retrovirus derivative was used in which the enhancer/promoter region of the 3' LTR was deleted. As a result, viral regulated transcription is generated only by the 5' LTR. Genes which are positioned in an anti-sense orientation to the 5' LTR will only be regulated by their own promoter [Hofmann, A., G.P. Nolan, and H.M. Blau, Proc Natl Acad Sci U S A, 1996. 93(11): p. 5185-90].

Preferably, all three modules of the system are encoded in a single retrovirus; otherwise it would be necessary to deliver it by three independent infections. This, however, might disrupt the regulation of the present system. Transcription of any element in the network (either the fusion proteins or the output) might generate a single mRNA molecule that contains other downstream elements. As a result, downstream elements might be regulated by an upstream promoter in addition to their own. In the plasmid system, this problem was easily avoided by a transcription terminator sequence placed downstream to each module of the network. However, the retroviral DNA must not contain any terminator sequence between the 5' LTR and the 3' LTR. Otherwise, transcription of retroviral RNA will be impaired and virion titer will be extremely reduced. Accordingly, efficient translation initiation from the middle of an mRNA molecule requires an Internal Ribosome Entry Site (IRES) [Hellen, C.U. and P. Sarnow, Genes Dev, 2001. 15(13): p. 1593-612].

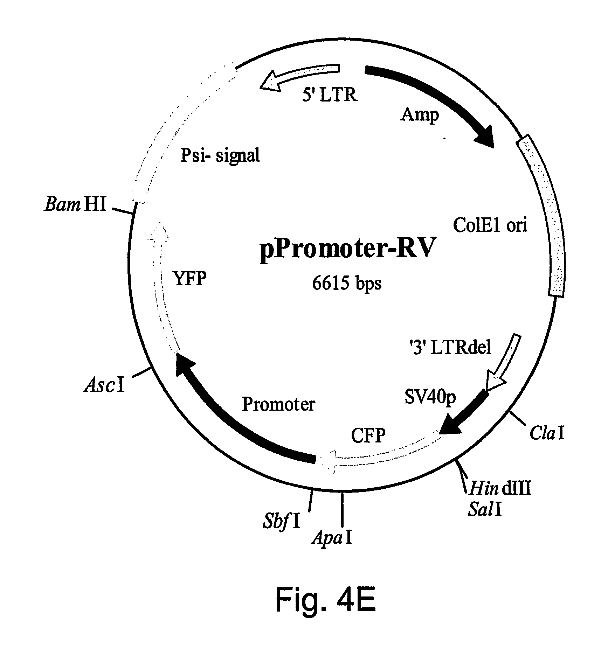

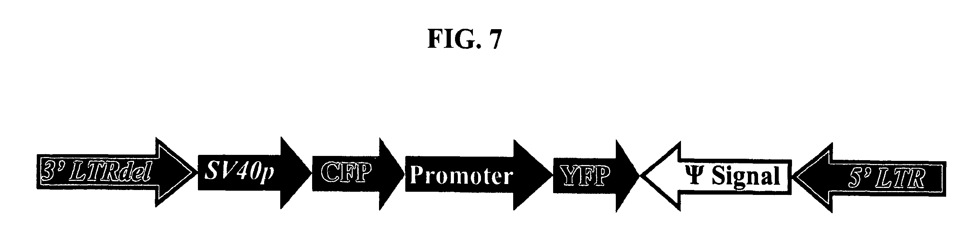

To examine whether transcription of an upstream element affects the regulation of downstream elements, a retrovirus was constructed in which CFP expression was regulated by an SV40 promoter. Immediately downstream to the CFP (which contained a 3' STOP codon) a YFP was cloned. This construct, together with subsequent constructs in which an SV40 or human promoters were cloned upstream to the YFP gene, were used for further analysis (plasmid pPromoter-RV, Figure 4E). A scheme of these retroviruses is described in Figure 7.

It may be expected that if the expression of upstream elements also regulates the expression of downstream elements, CFP and YFP would be co-expressed even in the absence of a promoter upstream to the YFP gene. Furthermore, if a human promoter would be placed upstream to the YFP gene, YFP concentrations would be significantly higher than those measured when YFP is regulated solely by the human promoter. This might occur if YFP was co-regulated by the potent SV40 promoter in addition to the human promoter.

### MATERIALS AND METHODS

CFP and YFP measurements were performed in the LSRII FACS machine. Infection of WI38/T/R/G cells using the Phoenix-Eco retroviral system was performed as described in [Nolan, G.P. and A.R. Shatzman, Curr Opin Biotechnol, 1998. 9(5): p. 447-50]. Fluorescence generated by the present system was first measured in the 293T cells which were used to produce the virions needed to infect the WI38 cells. Subsequently, following infection procedure, fluorescence was also measured in the WI38 cells.

### RESULTS

YFP expression was not affected by the 3'LTR. YFP expression was not affected by the activity of the SV40 promoter. When there was no promoter upstream to the YFP gene, fluorescence values which were very similar to the background fluorescence was measured. Furthermore, when promoters which show relatively low activity in 293T cells regulated YFP expression, YFP levels remained low as expected. Therefore, it can be concluded that the expression of upstream elements *probably* do not significantly affect the expression of downstream elements. Thus, it should be possible to include all the three modules of the network in a single retrovirus.

### EXAMPLE 6

### Cancer Detection using the constructs of the present invention

### MATERIALS AND METHODS

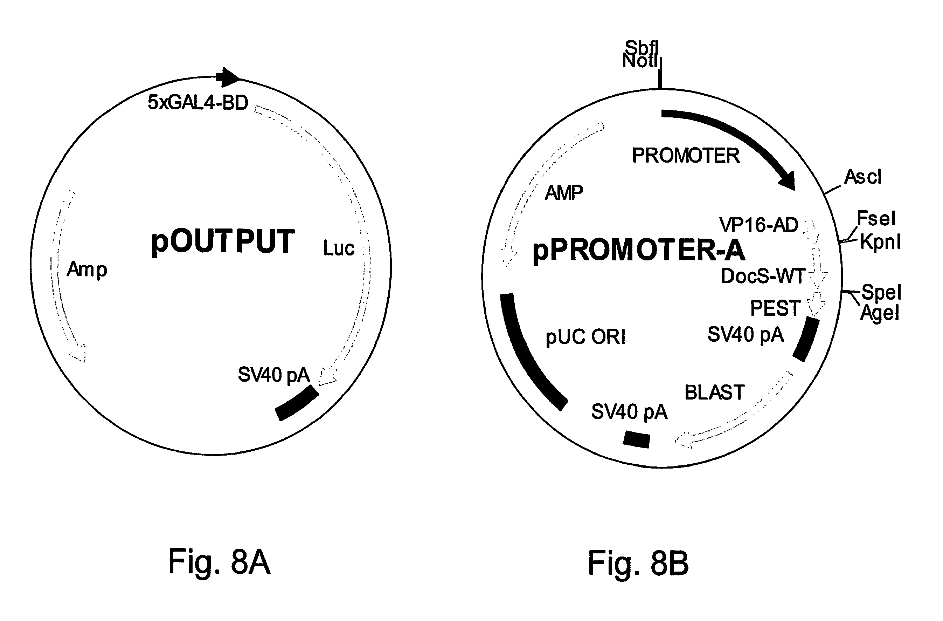

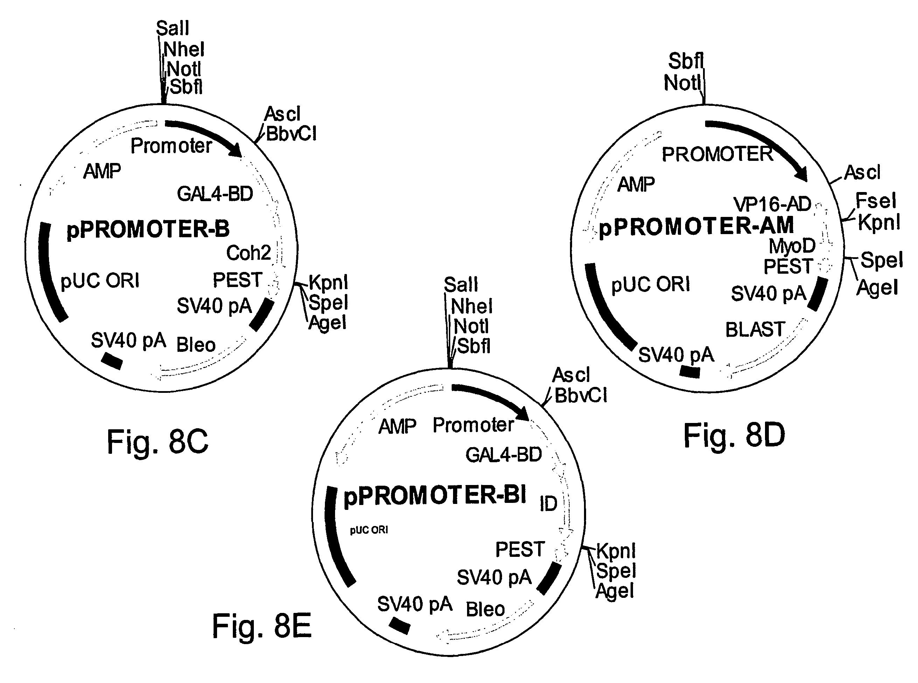

To examine the behavior of the two-promoter system cancer cells (293T), three plasmids were used for transfection: (1) pPROMOTER-A: human promoter regulating Activation Domain fused to DocS (Figure 8B); (2) pPROMOTER-B: human promoter regulating Binding Domain fused to Coh2 (Figure 8C); (3) pOUTPUT: promoter composed of five repeats of Yeast GAL4 binding sites regulating Luciferase output (Figure 8A).

As well as DocS and Coh2 as protein-protein interaction pairs, MyoD and ID [Davis, R.L., et al., Cell, 1987. 51(6): p. 987-1000; Benezra, R., et al., Cell, 1990. 61 (1): p. 49-59; Finkel, T., et al., J Biol Chem, 1993. 268(1): p. 5-8] taken from the commercial mammalian two-hybrid system (Promega CheckMate Cat #E2440) were also used.

Accordingly, the cells were transfected with the below three plasmids:
(1) pPROMOTER-AM: human promoter regulating Activation Domain fused to MyoD (Figure 8D); (2) pPROMOTER-BI: human promoter regulating Binding Domain fused to ID (Figure 8E); (3) pOUTPUT: promoter composed of five repeats of Yeast GAL4 binding sites regulating Luciferase output (Figure 8A).

In addition, to testing the mutual activity of two different promoters, the above experiment was repeated, wherein the two promoters were identical. This system could be used to distinguish between normal and aberrant over-expression of a single gene.

### RESULTS

As illustrated in Figures 9-11, output protein was expressed in cancer cells.

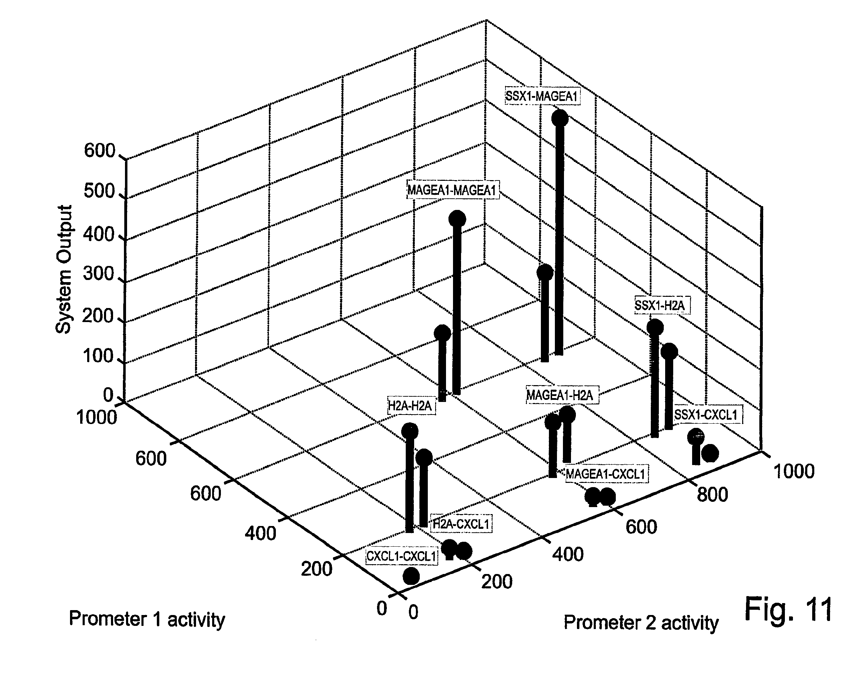

### Mutual activity of two different promoters - Figure 9 and Figure 11

When the weakest promoter (CXCL 1) was used with any of the others, the output was very low, irrespective of the second promoter's activity level.

With two input promoters having medium to high range activity (H2A with SSX1 or MAGEA1), the output was medium range too.

With both promoters highly active (MAGEA1 and SSX1) the output was very high for the DocS-Coh2 system but medium for the MyoD-ID system. DocS and Coh2 bind with a higher affinity than MyoD and ID. Thus, it would seem that stronger binding pairs enhance the sensitivity of response.

The above results prove that the present system can indeed identify cancer cells according to the activity pattern of their input promoters. In the present example, the system could identify a unique state characterized by high mutual activity of two input promoters and all other states.

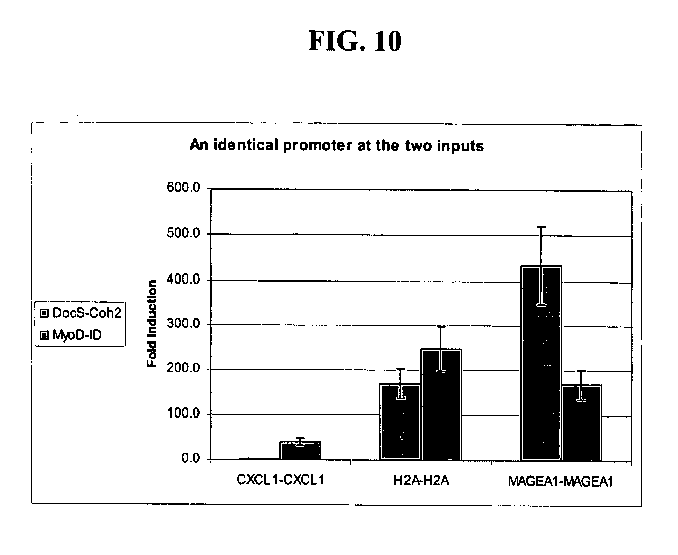

### Single promoter input - Figures 10 and 11

The weak promoters generated weak output (CXCL1-CXCL1) for both DocS-Coh2 and MyoD-ID

The medium promoters generated medium output (H2A-H2A) for both DocS-Coh2 and MyoD-ID

However, strong promoters generated very strong output with DocS-Coh2, but only medium output (MAGEA1-MAGEA1) with MyoD-ID.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. A nucleic acid construct system, comprising:
(i) first nucleic acid construct comprising a first polynucleotide, said first polynucleotide comprising a first nucleic acid sequence encoding a first expression product, said first nucleic acid sequence being operably linked to a first inducible mammalian transcriptional regulatory sequence; and
(ii) second nucleic acid construct comprising a second polynucleotide, said second polynucleotide comprising a second nucleic acid sequence encoding a second expression product, said second nucleic acid sequence being operably linked to a second inducible mammalian transcriptional regulatory sequence,
wherein said first inducible mammalian transcriptional regulatory sequence and said second inducible mammalian transcriptional regulatory sequence are non-identical and each independently selected from a cell cycle responsive mammalian transcriptional regulatory sequence.

2. The nucleic acid construct system of claim 1, further comprising a third nucleic acid construct comprising a third polynucleotide, said third polynucleotide comprising a third nucleic acid sequence encoding a reporter polypeptide, operably linked to a promoter, wherein an activity of said promoter is regulated by binding of at least one of said first and said second expression product.

3. The nucleic acid construct system of claim 2, wherein said reporter polypeptide comprises a detectable moiety.

4. The nucleic acid construct system of claim 2, wherein said reporter polypeptide comprises a therapeutic polypeptide.

5. The nucleic acid construct system of claim 2, wherein said first expression product and said second expression product are capable of binding to form a transcriptional regulator of said promoter,

6. The nucleic acid construct system of claim 2, wherein said first expression product is a polypeptide capable of activating said promoter.

7. The nucleic acid construct system of claim 6, wherein said second expression product is a polynucleotide capable of down-regulating an expression of said first expression product or said reporter polypeptide.

8. The nucleic acid construct system of claim 7, wherein said polynucleotide capable of down-regulating an expression of said first expression product is an RNA silencing oligonucleotide.

9. The nucleic acid construct system of claim 1, wherein said first polynucleotide and/or said second polynucleotide further comprises a nucleic acid sequence encoding a degradation tag.

10. The nucleic acid construct system of claim 3 for use in diagnosing a disease or a metabolic state of a mammalian cell, wherein a change in expression of said reporter polypeptide is indicative of the disease or metabolic state.

11. The nucleic acid construct system of claim 4 for use in treating a disease.

12. A method of identifying an agent capable of reversing a disease phenotype of a mammalian cell, the method comprising,
(a) ex vivo expressing the nucleic acid construct system of claim 3 in the mammalian cell;
(b) contacting the mammalian cell with the agent; and
(c) measuring a level of detectable moiety following (b) and optionally prior to (b), wherein a reversion of phenotype is indicative of an agent capable of reversing a diseased phenotype of a mammalian cell.

13. A nucleic acid construct, comprising:
(i) first polynucleotide, said first polynucleotide comprising a first nucleic acid sequence encoding a first expression product, said first nucleic acid sequence being operably linked to a first inducible mammalian transcriptional regulatory sequence; and
(ii) second polynucleotide comprising a second nucleic acid sequence encoding a second expression product, said second nucleic acid sequence being operably linked to a second inducible mammalian transcriptional regulatory sequence,
wherein said first inducible mammalian transcriptional regulatory sequence and said second inducible mammalian transcriptional regulatory sequence are non-identical and each independently selected from a cell cycle responsive mammalian transcriptional regulatory sequence.

14. The nucleic construct of claim 13, further comprising a third polynucleotide, said third polynucleotides comprising a third nucleic acid sequence encoding a reporter polypeptide, operably linked to a promoter, wherein an activity of said promoter is regulated by binding of at least one of said first and said second expression product.

## Patentansprüche

1. Nukleinsäurekonstruktsystem, umfassend:
i) ein erstes Nukleinsäurekonstrukt, umfassend ein erstes Polynukleotid, wobei das erste Polynukleotid eine erste Nukleinsäuresequenz umfasst, die für ein erstes Expressionsprodukt kodiert, wobei die erste Nukleinsäuresequenz mit einer ersten induzierbaren transkriptionellen regulatorischen Sequenz eines Säugers operativ verknüpft ist; und
ii) ein zweites Nukleinsäurekonstrukt, umfassend ein zweites Polynukleolid, wobei das zweite Polynukleotid eine zweite Nukleinsäuresequenz umfasst, die für ein zweites Expressionsprodukt kodiert, wobei die zweite Nukleinsäuresequenz mit einer zweiten induzierbaren transkriptionellen regulatorischen Sequenz eines Säugers operativ verknüpft ist,
wobei die erste induzierbare transkriptionette regulatorische Sequenz eines Säugers und die zweite induzierbare transkriptionelle regulatorische Sequenz eines Säugers nicht identisch sind und jeweils unabhängig aus einer auf den Zellzyklus reagierenden transkriptionellen regulatorischen Sequenz eines Säugers ausgewählt sind.

2. Nukleinsäurekonstruktsystem nach Anspruch 1, ferner umfassend ein drittes Nukleinsäurekonstrukt, umfassend ein drittes Polynukleotid, wobei das dritte Polynukleotid eine dritte Nukleinsäuresequenz umfasst, die für ein Reporter-Polypeptid kodiert, operativ verknüpft mit einem Promotor, wobei eine Aktivität des Promotors durch das Binden zumindest eines des ersten und des zweiten Expt-essionspr-odukts reguliert wird.

3. Nukleinsäurekonstruktsystem nach Anspruch 2, wobei das Reporter-Polypeptid eine nachweisbare Einheit umfasst.

4. Nukleinsautekonstruktsystem nach Anspruch 2, wobei das Reporter-Polypeptid ein therapeutisches Polypeptid umfasst.

5. Nukleinsäurekonstruktsystem nach Anspruch 2, wobei das erste Expressionsprodukt und das zweite Expressionsprodukt zur Bindung in der Lage sind, um einen Transkriptionsregulator des Promotors zu bilden,

6. Nukleinsäurekonstruktsystem nach Anspruch 2, wobei das erste Expressionsprodukt ein Polypeptid ist, das in der Lage ist, den Promotor zu aktivieren.

7. Nukleinsäurekonstruktsystem nach Anspruch 6, wobei das zweite Expressionsprodukt ein Polynukleotid ist, das in der Lage ist, eine Expression des ersten Expressionsprodukts oder des Reporter-Polypeptids herunterzuregulieren.

8. Nukleinsäurekonstruktsystem nach Anspruch 7, wobei das Polynukleotid, das in der Lage ist, eine Expression des ersten Expressionsprodukts herunterzuregulieren, ein RNA-Sileneing-Oligonukleotid ist.

9. Nukleinsäurekonstruktsystem nach Anspruch 1, wobei das erste Polynukleotid und/oder das zweite Polynukleotid ferner eine Nukleinsäuresequenz umfasst bzw. umfassen, die für ein Degradations-Tag kodieren.

10. Nukleinsäurekonstruktsystem nach Anspruch 3 zur Verwendung bei der Diagnose einer Krankheit oder eines metabolischen Zustands einer Säugerzelle, wobei eine Veränderung der Expression des Reporter-Polypeptids für die Krankheit oder den metabolische Zustand indikativ ist.

11. Nukleinsäurekonstruktsystem nach Anspruch 4 zur Verwendung bei der Behandlung einer Krankheit.

12. Verfahren zum Identifizieren eines Agens, das in der Lage ist, einen Krankheitsphänotypen einer Säugerzelle umzukehren, wobei das Verfahren umfasst:
a) Ex-vivo-Exprimieren des Nukleinsäurekonstruktsystems nach Anspruch 3 in der Säugerzelle;
b) Kontaktierten der Säugerzelle mit dem Agens; und
c) Messen einer Menge an nachweisbarer Einheit nach (b) und optional vor (b), wobei eine Umkehr des Phänotypen für ein Agens indikativ ist, das in der Lage ist, einen Krankheitsphänotypen einer Säugerzelle umzukehren.

13. Nukleinsäurekonstrukt, umfassend:
i) ein erstes Polynukleotid, wobei das erste Polynukleotid eine erste Nukleinsäuresequenz umfasst, die für ein erstes Expressionsprodukt kodiert, wobei die erste Nukleinsäuresequenz mit einer ersten induzierbaren transkriptionellen regulatorischen Sequenz eines Säugers operativ verknüpft ist; und
ii) ein zweites Polynukleotid, das eine zweite Nukleinsäuresequenz umfasst, die für ein zweites Expressionsprodukt kodiert, wobei die zweite Nukleinsäuresequenz mit einer zweiten induzierbaren transkriptionellen regulatorischen Sequenz eines Säugers operativ verknüpft ist,
wobei die erste induzierbare transkriptionelle regulatorische Sequenz eines Säugers und die zweite induzierbare transkriptionelle regulatorische Sequenz eines Säugers nicht identisch sind und jeweils unabhängig aus einer auf den Zellzyklus reagierenden transkriptionellen regulatorischen Sequenz eines Säugers ausgewählt sind,

14. Nukleinsäurekonstrukt nach Anspruch 13, ferner umfassend ein drittes Polynukleotid, wobei das dritte Polynukleotid eine dritte Tqukleinsäuresequenz umfasst, die für ein Reporter-Polypeptid kopiert, operativ verknüpft mit einem Promotor, wobei eine Aktivität des Promotors durch das Binden zumindest eines des ersten und des zweiten Expressionsprodukts reguliert wird,

## Revendications

1. Système de produit de recombinaison d'acide nucléique, comprenant :
(i) un premier produit de recombinaison d'acide nucléique comprenant un premier polynucléotide, ledit premier polynucléotide comprenant une première séquence d'acide nucléique codant pour un premier produit d'expression, ladite première séquence d'acide nucléique étant en liaison fonctionnelle avec une première séquence de régulation de la transcription de mammifère inductible ; et
(ii) un deuxième produit de recombinaison d'acide nucléique comprenant un deuxième polynucléotide, ledit deuxième polynucléotide comprenant une deuxième séquence d'acide nucléique codant pour un deuxième produit d'expression, ladite deuxième séquence d'acide nucléique étant en liaison fonctionnelle avec une deuxième séquence de régulation de la transcription de mammifère inductible,
où ladite première séquence de régulation de la transcription de mammifère inductible et ladite deuxième séquence de régulation de la transcription de mammifère inductible ne sont pas identiques et sont chacune indépendamment sélectionnées à partir d'une séquence de régulation de la transcription de mammifère sensible au cycle cellulaire.

2. Système de produit de recombinaison d'acide nucléique selon la revendication 1, comprenant en outre un troisième produit de recombinaison d'acide nucléique comprenant un troisième polynucléotide, ledit troisième polynucléotide comprenant une troisième séquence d'acide nucléique codant pour un polypeptide rapporteur, en liaison fonctionnelle avec un promoteur, où une activité dudit promoteur est régulée par la liaison d' au moins un dudit premier et dudit deuxième produit d'expression.

3. Système de produit de recombinaison d'acide nucléique selon la revendication 2, dans lequel ledit polypeptide rapporteur comprend un fragment détectable.

4. Système de produit de recombinaison d'acide nucléique selon la revendication 2, dans lequel ledit polypeptide rapporteur comprend un polypeptide thérapeutique.

5. Système de produit de recombinaison d'acide nucléique selon la revendication 2, dans lequel ledit premier produit d'expression et ledit deuxième produit d'expression sont capables de se lier pour former un régulateur de la transcription dudit promoteur.

6. Système de produit de recombinaison d'acide nucléique selon à revendication 2, dans lequel ledit premier produit d'expression est un polypeptide capable d'activer ledit promoteur.

7. Système de produit de recombinaison d'acide nucléique selon la revendication 6, dans lequel ledit deuxième produit d'expression est un polynucléotide capable de négativement réguler une expression dudit premier produit d'expression ou dudit polypeptide rapporteur.

8. Système de produit de recombinaison d'acide nucléique selon la revendication 7, dans lequel ledit polynucléotide capable de négativement réguler une expression dudit premier produit d'expression est un oligonucléotide d'extinction de l'ARN.

9. Système de produit de recombinaison d'acide nucléique selon la revendication 1, dans lequel ledit premier polynucléotide et/ou ledit deuxième polynucléotide comprend en outre une séquence d'acide nucléique codant pour un marqueur de dégradation.

10. Système de produit de recombinaison d'acide nucléique selon la revendication 3 destiné à être utilisé dans le diagnostic d'une maladie ou d' un état métabolique d'une cellule de mammifère, dans lequel une modification de l'expression dudit polypeptide rapporteur indique la maladie ou l'état métabolique.

11. Système de produit de recombinaison d'acide nucléique selon la revendication 9, destiné à être utilisé dans le traitement d'une maladie.

12. Procédé pour identifier un agent capable d'inverser un phénotype pathologique d'une cellule de mammifère, le procédé consistant à,
(a) exprimer ex vivo le système de produit de recombinaison d'acide nucléique selon la revendication 3 dans la cellule de mammifère ;
(b) mettre en contact la cellule de mammifère avec l'agents et
(c) mesurer un taux de fragment détectable après (b) et facultativement avant (b), où une inversion de phénotype indique un agent capable d'inverser un phénotype pathologique d'une cellule de mammifère.

13. Produit de recombinaison d'acide nucléique, comprenant :
(i) un premier polynucléotide, ledit premier polynucléotide comprenant une première séquence d'acide nucléique codant pour un premier produit d'expression, ladite première séquence d'acide nucléique étant en liaison fonctionnelle avec une première séquence de régulation de la transcription de mammifère inductible ; et
(ii) un deuxième polynucléotide comprenant une deuxième séquence d'acide nucléique codant pour un deuxième produit d'expression, ladite deuxième séquence d'acide nucléique étant en liaison fonctionnelle avec une deuxième séquence de régulation de la transcription de mammifère inductible,
où ladite première séquence de régulation de la transcription de mammifère inductible et ladite deuxième séquence de régulation de la transcription de mammifère inductible ne sont pas identiques et sont chacune indépendamment sélectionnées à partir d'une séquence de régulation de la transcription de mammifère sensible au cycle cellulaire.

14. Produit de recombinaison d'acide nucléique selon la revendication 13, comprenant en outre un troisième polynucléotide, ledit troisième polynucléotide comprenant une troisième séquence d'acide nucléique codant pour un polypeptide rapporteur, en liaison fonctionnelle avec un promoteur, où une activité dudit promoteur est régulée par la liaison d'au moins un dudit premier et dudit deuxième produit d'expression.
